# EUROPEAN PATENT APPLICATION

(11) **EP 4 778 551 A1**
(43) Date of publication of application: **22.07.2026**
(21) Application number: 24882088.8
(22) Date of filing: 25.09.2024
(51) Int. Cl.: A61M 5/20

(54) **DRUG SOLUTION ADMINISTRATION DEVICE, INTERMEDIATE ASSEMBLY OF DRUG SOLUTION ADMINISTRATION DEVICE, METHOD FOR ASSEMBLING DRUG SOLUTION ADMINISTRATION DEVICE, AND MEDICAL INSTRUMENT PACKAGE**

(30) Priority: 26.10.2023 JP 2023183872
(71) Applicant: TERUMO Kabushiki Kaisha, Tokyo 151-0072 (JP)
(72) Inventor: OKIHARA, Hitoshi, Fujinomiya-shi, Shizuoka 418-0004 (JP)
(74) Representative: Prüfer & Partner mbB Patentanwälte · Rechtsanwälte
(86) International application number: PCT/JP2024/034177
(87) International publication number: WO 2025/088971

(57) **Abstract**

An intermediate assembly (Sa) of a liquid medicine administration device (10) includes a first unit (U1) and a second unit (U2) attached to the first unit (U1). The first unit (U1) has a hollow cylindrical housing (12). The second unit (U2) has a drive unit (D) and is attached to a fixing portion (27) of the housing (12) of the first unit (U1). In a state where a syringe (16) is not housed in the first unit (U1), provisional fixing of the second unit (U2) to the fixing portion (27) of the first unit (U1) is performed. In a state where the second unit (U2) is detached from the first unit (U1) so that a provisional fixing state is released, and the syringe (16) is housed in the housing (12) of the first unit (U1), main fixing of the second unit (U2) and the first unit (U1) is able to be performed.

## Description

### Technical Field

The present invention relates to a liquid medicine administration device, an intermediate assembly of the liquid medicine administration device, a method for assembling the liquid medicine administration device, and a medical instrument package.

### Background Art

JP 6334717 B2 discloses a liquid medicine administration device that includes a cylindrical casing, a trigger member as a needle cover slidably provided on a distal end of the casing, and a syringe housed in the casing and filled with a liquid medicine, and that is capable of administering the liquid medicine in the syringe by the trigger member being operated.

### Patent Literature

Patent Literature 1: JP 6334717 B2

### Summary of Invention

In the liquid medicine administration device, simplification of an assembly step is desired.

An object of the present invention is to solve the above-described problem.
(1) According to a first aspect of the present invention, there is provided an intermediate assembly of a liquid medicine administration device, including: a first unit; and a second unit attached to the first unit. The first unit has a hollow cylindrical housing and enables a syringe filled with a liquid medicine to be housed in the housing. The second unit has a drive unit biasing a gasket of the syringe toward a distal end of the housing and is attached to a fixing portion provided at a proximal portion of the housing of the first unit, and at least a part of the second unit is housed in the housing. In a state where the syringe is not housed in the first unit, provisional fixing of the second unit to the fixing portion of the first unit is performed. In a state where the second unit is detached from the fixing portion of the first unit so that a provisional fixing state is released, and the syringe is housed in the housing of the first unit, main fixing of the second unit and the first unit is able to be performed.
   According to the intermediate assembly of a liquid medicine administration device, the number of units supplied in an assembly step can be reduced by using the intermediate assembly in which the first and second units are provisionally fixed, and an assembly step of the liquid medicine administration device can be simplified, as compared with a configuration in which a first member, a second member, and a syringe filled with a liquid medicine form respective units, and the three units are finally assembled.
(2) In the intermediate assembly according to (1), the second unit may have a first engagement portion for provisional fixing and a second engagement portion for main fixing. In a state where the first unit and the second unit are provisionally fixed, the first engagement portion may be engaged with the fixing portion. In a state where the provisional fixing state between the first unit and the second unit is released, and main fixing of the first unit and the second unit is completed, the first engagement portion may be disengaged from the fixing portion, and the second engagement portion may be engaged with the fixing portion.
   With this configuration, provisional fixing of the first unit and the second unit can be performed by engaging the first engagement portion with the fixing portion, and in a state where the syringe is housed, main fixing of the first unit and the second unit can be effectively performed by engaging the second engagement portion with the fixing portion.
(3) In the intermediate assembly according to (2), the fixing portion may include a provisional fixing portion and a main fixing portion provided at a position different from a position of the provisional fixing portion. In a state where the first unit and the second unit are provisionally fixed, the first engagement portion may be engaged with the provisional fixing portion. In a state where main fixing of the first unit and the second unit is completed, the first engagement portion may be disengaged from the provisional fixing portion, and the second engagement portion may be engaged with the main fixing portion.
   With this configuration, provisional fixing of the first unit and the second unit can be performed by engaging the first engagement portion with the provisional fixing portion, and in the state where the syringe is housed, main fixing of the first unit and the second unit can be effectively performed by engaging the second engagement portion with the main fixing portion.
(4) In the intermediate assembly according to (3), the fixing portion may be a hole or a recess formed in the housing. The provisional fixing portion and the main fixing portion may be formed in different areas of the hole or the recess.
   With this configuration, the provisional fixing portion and the main fixing portion can be provided in the same hole or recess, so that the provisional fixing portion and the main fixing portion can be easily formed.
(5) In the intermediate assembly according to (3) or (4), the main fixing portion may be provided on a proximal side of the housing in an axial direction with respect to the provisional fixing portion.
   With this configuration, by moving the second unit in the distal direction, the first engagement portion can be disengaged from the provisional fixing portion in a distal direction, and the second engagement portion can be engaged with the main fixing portion.
(6) In the intermediate assembly according to (5), the fixing portion may have a first region in which the provisional fixing portion is provided and a second region which is adjacent to a proximal side of the first region in the axial direction of the housing and in which the main fixing portion is provided. The first region may be formed to be wider than the second region in a circumferential direction of the housing.
   With this configuration, the provisional fixing portion and the main fixing portion can be easily provided in one hole or recess.
(7) In the intermediate assembly according to any one of (2) to (6), the fixing portion may be a hole or a recess formed in the housing. The first engagement portion may have a first engagement protrusion that is provided on an outer circumferential surface of a sleeve body and is engageable with the fixing portion, the sleeve body being attached to the proximal portion of the housing and being housed in the housing. The second engagement portion may have a second engagement protrusion that is provided on an end cap attached to a proximal end of the sleeve body and is engageable with the fixing portion.
   With this configuration, provisional fixing of the first unit and the second unit can be performed by engaging the first engagement protrusion provided on the sleeve body with the fixing portion, and main fixing of the first unit and the second unit can be effectively performed by engaging the second engagement protrusion provided on the end cap with the fixing portion.
(8) In the intermediate assembly according to any one of (2) to (7), a plurality of the fixing portions may be provided in the circumferential direction of the housing. A plurality of the first engagement portions and a plurality of the second engagement portions may be individually provided in a circumferential direction of the second unit.
   With this configuration, when the second unit is inserted into and provisionally fixed to the first unit, the first engagement portion and the second engagement portion are easily aligned with the fixing portion in the circumferential direction of the housing and the second unit.
(9) In the intermediate assembly according to any one of (3) to (6), the housing may have a reception portion formed in a hole or a recess located in a distal direction with respect to the fixing portion. The first engagement portion may be inserted into the reception portion when the first unit and the second unit are fixed by the main fixing portion.
   With this configuration, in a state where the first unit and the second unit are fixed, the first engagement portion moved in the distal direction from the fixing portion is inserted into the reception portion, so that interference between the first engagement portion and the housing is effectively prevented.
(10) According to a second aspect of the present invention, there is provided a liquid medicine administration device including: a first unit having a hollow cylindrical housing; a syringe housed in the housing and filled with a liquid medicine; and a second unit which has a drive unit biasing a gasket of the syringe toward a distal end of the housing and is attached to the first unit, and at least a part of which is housed in the housing. A proximal portion of the housing of the first unit has a fixing portion. The second unit has a first engagement portion for provisional fixing and a second engagement portion for main fixing. The first engagement portion is an engagement portion used to provisionally fix the second unit to the fixing portion of the first unit by being engaged with the fixing portion before the syringe is housed in the housing of the first unit. In an assembly completed state where the syringe is housed in the housing, and main fixing of the second unit to the fixing portion of the first unit is completed, the first engagement portion is disengaged from the fixing portion, and the second engagement portion is engaged with the fixing portion.
   According to the liquid medicine administration device, the number of units supplied in an assembly step can be reduced by using the intermediate assembly in which the first and second units are provisionally fixed, and an assembly step of the liquid medicine administration device can be simplified, as compared with a configuration in which a first member, a second member, and a syringe filled with a liquid medicine form respective units, and the three units are finally assembled.
(11) According to a third aspect of the present invention, there is provided a method for assembling a liquid medicine administration device using the intermediate assembly according to (1), the method including: a first step of detaching the second unit from the fixing portion of the first unit of the intermediate assembly and disengaging the second unit from the first unit in a proximal direction; a second step of inserting the syringe into the housing in a distal direction from the proximal portion of the first unit and housing the syringe in the housing; and a third step of inserting the second unit into the housing from the proximal portion of the first unit and performing main fixing of the second unit to the fixing portion of the first unit.
   According to the method for assembling a liquid medicine administration device, the number of components supplied in an assembly step can be reduced by using the intermediate assembly in which the first and second units are provisionally fixed, and an assembly step of the liquid medicine administration device can be simplified, as compared with a configuration in which a first member, a second member, and a syringe filled with a liquid medicine form respective units, and the three units are finally assembled.
(12) In the method for assembling a liquid medicine administration device according to (11), the second unit may have a first engagement portion for provisional fixing and a second engagement portion for main fixing. In the first step, the second unit may be detached, from a provisional fixing state where the first engagement portion is engaged with the fixing portion, by disengaging the first engagement portion from the fixing portion. In the third step, the second unit may be brought into a main fixing state with respect to the first unit by engaging the second engagement portion with the fixing portion.
   With this method, provisional fixing of the first unit and the second unit can be performed by engaging the first engagement portion with the fixing portion, and main fixing of the first unit and the second unit can be effectively performed in a state where the syringe is housed by engaging the second engagement portion with the fixing portion.
(13) According to a fourth aspect of the present invention, there is provided a medical instrument package including: the intermediate assembly of a liquid medicine administration device according to any one of (1) to (9); and a packaging vessel in which the intermediate assembly is housed.

According to this medical instrument package, the intermediate assembly in which the first and second units are provisionally fixed can be integrally housed in the packaging vessel, as compared with a case where each of the first unit and the second unit is individually housed in the packaging vessel. Therefore, space saving of a storage place of the intermediate assembly can be achieved.

According to the present invention, provisional fixing of the second unit to the fixing portion of the first unit is performed in a state where the syringe is not housed in the first unit, and main fixing of the second unit and the first unit is able to be performed in a state where the second unit is detached from the first unit so that the provisional fixing state is released, and the syringe is housed in the first unit. Therefore, as compared with a case where the first member, the second member, and the syringe filled with a liquid medicine form respective units (assemblies), and the three assemblies are provided in a final assembly step, the number of assemblies to be supplied in the final assembly step can be reduced by using one intermediate assembly in which the first and second units are provisionally fixed, and an assembly step of the liquid medicine administration device can be simplified.

### Brief Description of Drawings

[Fig. 1] Fig. 1 is an external perspective view of a liquid medicine administration device according to an embodiment of the present invention.
[Fig. 2] Fig. 2 is an exploded perspective view of first and second units and a syringe of the liquid medicine administration device illustrated in Fig. 1.
[Fig. 3] Fig. 3 is an external perspective view of an intermediate assembly including the first and second units illustrated in Fig. 2.
[Fig. 4] Fig. 4 is an exploded perspective view of the liquid medicine administration device illustrated in Fig. 1.
[Fig. 5] Fig. 5 is an overall cross-sectional view of the liquid medicine administration device illustrated in Fig. 1.
[Fig. 6] Fig. 6 is an enlarged plan view illustrating a proximal portion of the liquid medicine administration device.
[Fig. 7] Fig. 7A is a cross-sectional view taken along line VIIA-VIIA in Fig. 6. Fig. 7B is a cross-sectional view taken along line VIIB-VIIB in Fig. 6.
[Fig. 8] Fig. 8 is an enlarged cross-sectional view illustrating a distal end and its neighboring parts of the liquid medicine administration device illustrated in Fig. 5.
[Fig. 9] Fig. 9 is a partially omitted perspective view of the liquid medicine administration device illustrated in Fig. 1.
[Fig. 10] Fig. 10 is an exploded perspective view illustrating a state where a drive unit is disassembled from a housing of the first unit.
[Fig. 11] Fig. 11 is an enlarged front view of the coupling mechanism and its neighboring parts.
[Fig. 12] Fig. 12 is an enlarged cross-sectional view illustrating a state where a proximal end of a holding arm of a cap tilts radially outward.
[Fig. 13] Fig. 13 is an overall cross-sectional view illustrating another cross section of the liquid medicine administration device.
[Fig. 14] Fig. 14 is an exploded perspective view of a drive unit.
[Fig. 15] Fig. 15 is a partially omitted perspective view illustrating a proximal end and its neighboring parts of a sleeve body.
[Fig. 16] Fig. 16 is a cross-sectional view taken along line XVI-XVI in Fig. 6.
[Fig. 17] Fig. 17 is an enlarged plan view illustrating a proximal portion of the intermediate assembly illustrated in Fig. 3.
[Fig. 18] Fig. 18 is a partially omitted cross-sectional view of the liquid medicine administration device.
[Fig. 19] Fig. 19 is an enlarged cross-sectional view illustrating a proximal end and its neighboring parts of the liquid medicine administration device illustrated in Fig. 13.
[Fig. 20] Fig. 20 is an enlarged perspective view illustrating a first connection portion and its neighboring parts in the liquid medicine administration device illustrated in Fig. 10.
[Fig. 21] Fig. 21A is a partially cutaway enlarged perspective view illustrating a state where a proximal end of a plunger is inserted into a guide groove of an end guide. Fig. 21B is an illustrative view of first operation at a distal end and its neighboring parts of the plunger.
[Fig. 22] Fig. 22A is a partially cutaway enlarged perspective view illustrating a state where the plunger starts to rotate along an inclined guide portion. Fig. 22B is an illustrative view of second operation at the distal end and its neighboring parts of the plunger.
[Fig. 23] Fig. 23 is an exploded perspective view of the intermediate assembly illustrated in Fig. 3.
[Fig. 24] Fig. 24 is a cross-sectional view taken along line XXIV-XXIV in Fig. 17.
[Fig. 25] Fig. 25 is a cross-sectional view taken along line XXV-XXV in Fig. 17.
[Fig. 26] Fig. 26 is a plan view of a medical instrument package containing the intermediate assembly.
[Fig. 27] Fig. 27 is a first illustrative view of assembling of the first connection portion and a second connection portion of the coupling mechanism.
[Fig. 28] Fig. 28 is a second illustrative view of assembling of the first connection portion and the second connection portion of the coupling mechanism.
[Fig. 29] Fig. 29 is an illustrative view of a state where the cap is pulled in a distal direction with respect to the housing.
[Fig. 30] Fig. 30 is an overall cross-sectional view illustrating a state where the cap is detached from the liquid medicine administration device.
[Fig. 31] Fig. 31 is an enlarged cross-sectional view illustrating the distal end and its neighboring parts of the liquid medicine administration device illustrated in Fig. 30.
[Fig. 32] Fig. 32 is an enlarged cross-sectional view illustrating the proximal end and its neighboring parts of the liquid medicine administration device illustrated in Fig. 30.
[Fig. 33] Fig. 33 is an overall cross-sectional view illustrating a state where the liquid medicine administration device illustrated in Fig. 30 starts skin puncture.
[Fig. 34] Fig. 34 is an overall cross-sectional view illustrating a state where the liquid medicine administration device illustrated in Fig. 33 ends the skin puncture.
[Fig. 35] Fig. 35 is an enlarged cross-sectional view illustrating the proximal end and its neighboring parts of the liquid medicine administration device illustrated in Fig. 33.
[Fig. 36] Fig. 36 is an enlarged cross-sectional view illustrating the proximal end and its neighboring parts of the liquid medicine administration device illustrated in Fig. 34.
[Fig. 37] Fig. 37 is an enlarged cross-sectional view illustrating the proximal end and its neighboring parts of the liquid medicine administration device.
[Fig. 38] Fig. 38 is an enlarged cross-sectional view illustrating the proximal end and its neighboring parts of the liquid medicine administration device.
[Fig. 39] Fig. 39 is an overall cross-sectional view illustrating the liquid medicine administration device in a state where liquid medicine administration is ended.
[Fig. 40] Fig. 40 is an overall cross-sectional view illustrating another cross section of the liquid medicine administration device after the liquid medicine administration is ended.
[Fig. 41] Fig. 41A is a partially cutaway enlarged perspective view illustrating a state where the plunger moves in the distal direction along a straight guide portion. Fig. 41B is an illustrative view of third operation at the distal end and its neighboring parts of the plunger.
[Fig. 42] Fig. 42 is an overall cross-sectional view illustrating the liquid medicine administration device in a state where a puncture needle is housed in a needle cover.
[Fig. 43] Fig. 43 is an enlarged cross-sectional view illustrating the proximal end and its neighboring parts of the liquid medicine administration device illustrated in Fig. 42.

### Description of Embodiments

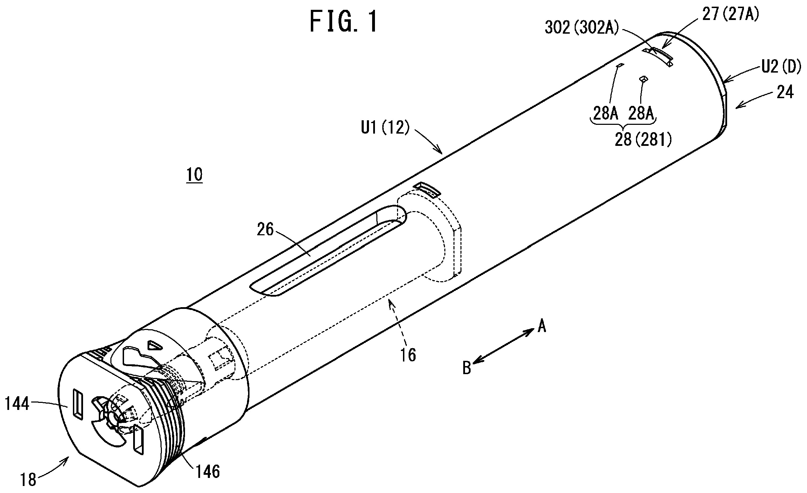

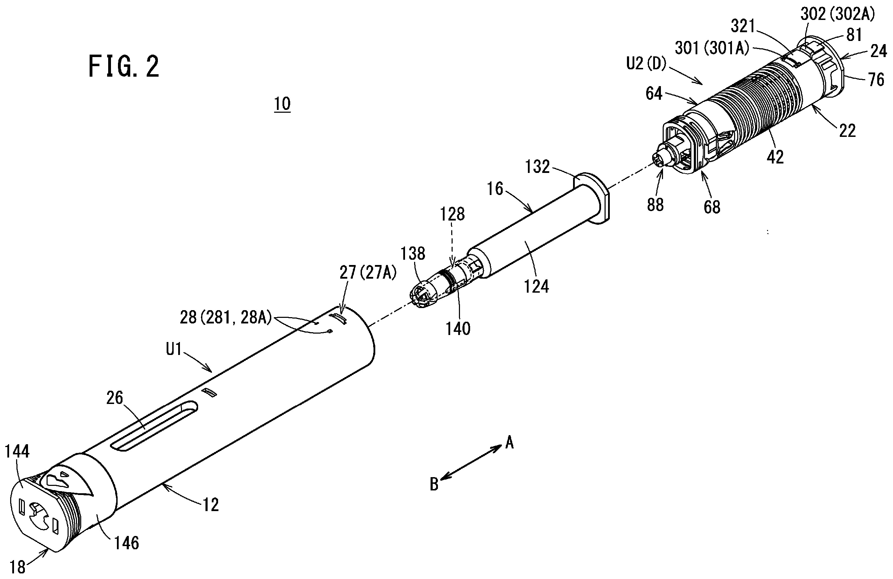

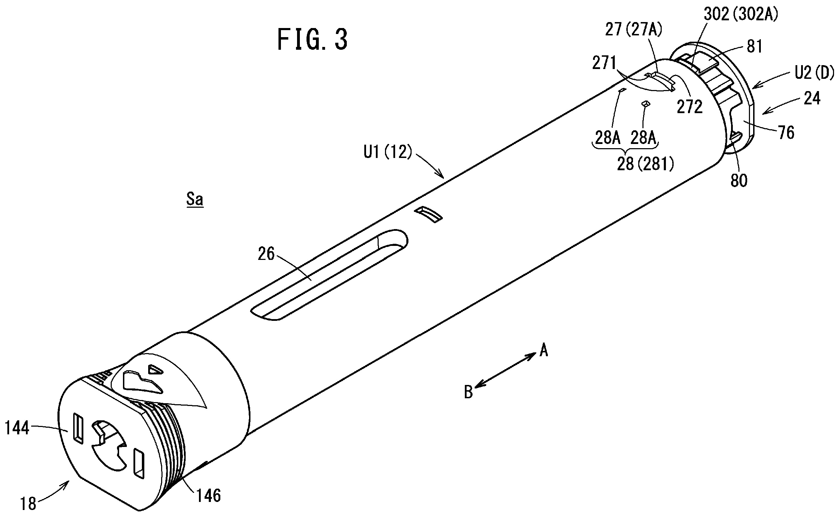

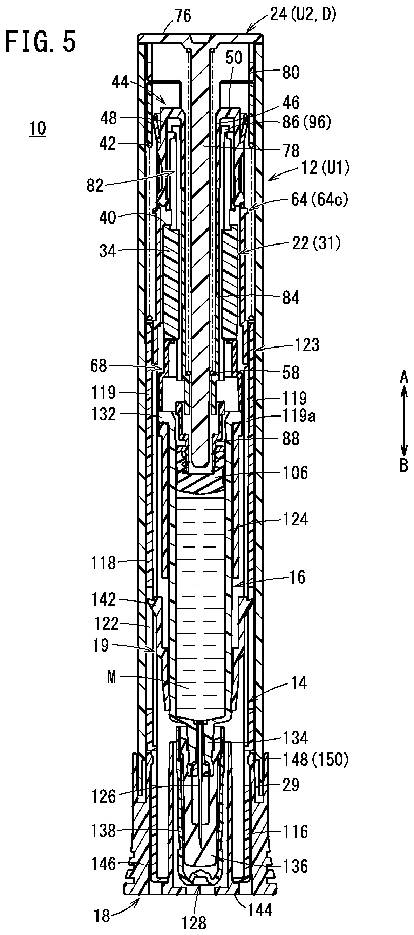

As illustrated in Fig. 1, a liquid medicine administration device 10 according to the embodiment is used to administer a liquid medicine M (see Fig. 5) subcutaneously to a patient who is a user. As illustrated in Fig. 2, the liquid medicine administration device 10 includes a first unit U1, a second unit U2 attached to the first unit U1, and a syringe 16 housed in the first unit U1 and filled with the liquid medicine M. In a process of assembling the liquid medicine administration device 10, an intermediate assembly Sa that is a provisional assembly in which the first unit U1 and the second unit U2 are provisionally fixed is formed (see Fig. 3).

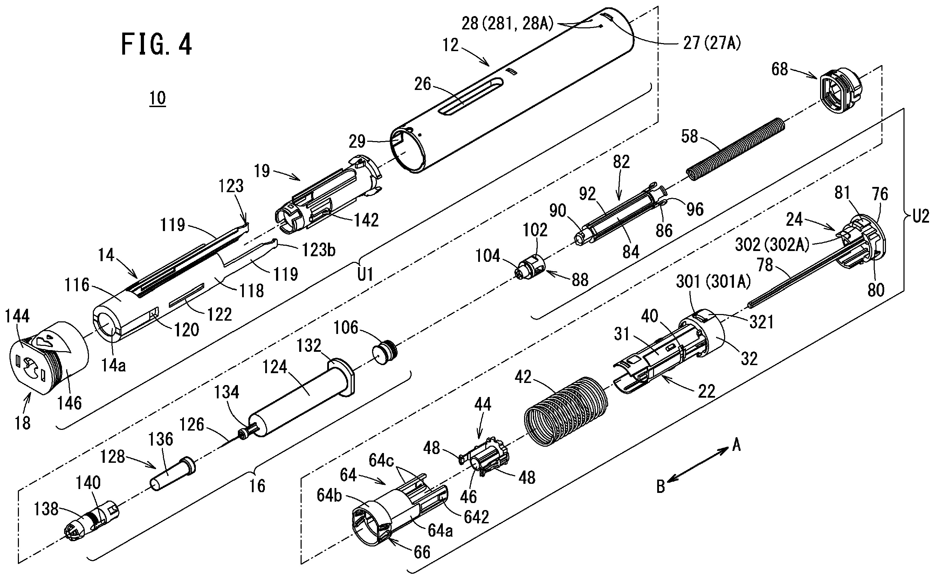

As illustrated in Fig. 4, the first unit U1 includes a hollow cylindrical housing 12, a needle cover 14 that is movably housed in the housing 12, a cap 18 that is detachably attached to a distal end of the housing 12, and a syringe holder 19 capable of holding the syringe 16.

The housing 12 extends along an axial direction (directions of arrows A and B). The housing 12 has distal and proximal ends which are individually open. As illustrated in Fig. 1, a circumferential wall of the housing 12 includes a viewing window 26, a fixing portion 27, and a reception portion 28. The viewing window 26 penetrates the circumferential wall of the housing 12 and is open at a position corresponding to a position of a barrel 124 of the syringe 16 housed in the housing 12. The liquid medicine M in the barrel 124 (see Fig. 2) can be checked through the viewing window 26.

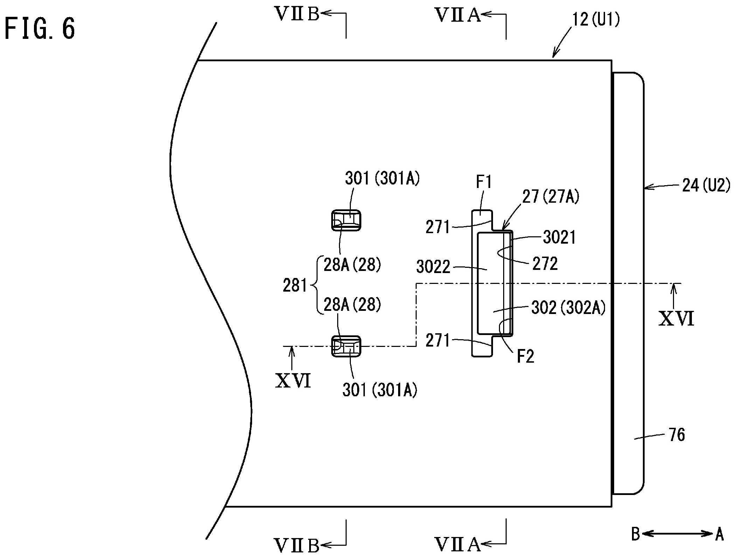

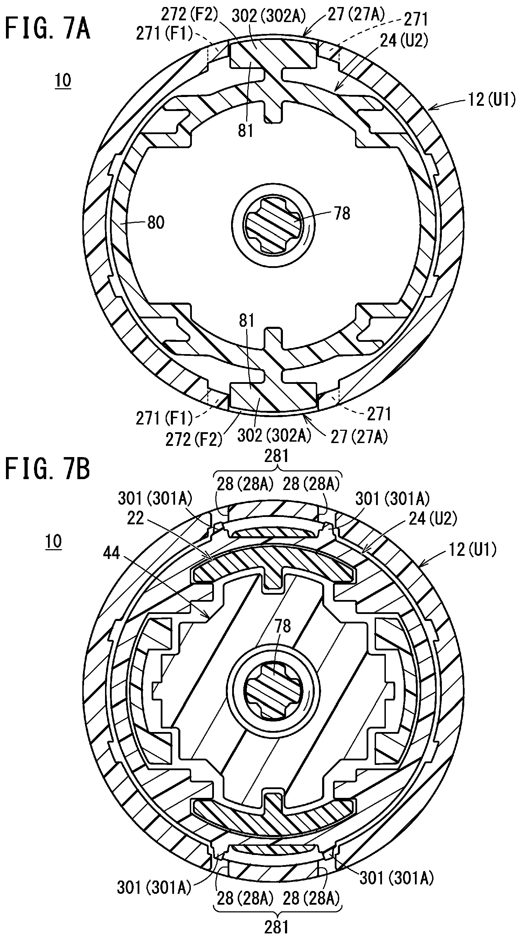

As illustrated in Fig. 6, the fixing portion 27 is provided at a proximal portion of the housing 12. As illustrated in Fig. 7A, a pair of fixing portions 27 are provided in a circumferential direction of the housing 12. The pair of fixing portions 27 face each other with a central axis of the housing 12 interposed therebetween. The pair of fixing portions 27 may not be located at positions facing each other with the central axis of the housing 12 interposed therebetween. Note that the present invention is not limited to the case where the pair of fixing portions 27 are provided. One or three or more fixing portions 27 may be provided. The fixing portion 27 is a first hole 27A penetrating the circumferential wall of the housing 12. As illustrated in Fig. 6, the fixing portion 27 has a shape elongated in the circumferential direction with respect to the axial direction of the housing 12.

The fixing portion 27 includes a pair of provisional fixing portions 271 and a main fixing portion 272. The first hole 27A of the fixing portion 27 has a first region F1 in which the provisional fixing portions 271 are provided and a second region F2 which is adjacent to a proximal side of the first region F1 and in which the main fixing portion 272 is provided. In the circumferential direction of the housing 12, the first region F1 is formed to be wider than the second region F2. In other words, in the axial direction of the housing 12, the provisional fixing portion 271 and the main fixing portion 272 are provided at different positions, and the main fixing portion 272 is located on a proximal side (the direction of the arrow A) with respect to the provisional fixing portion 271. The pair of provisional fixing portions 271 are located at both ends, respectively, in a width direction of the first region F1. The two provisional fixing portions 271 project equally in the circumferential direction from both ends of the main fixing portion 272 in the circumferential direction.

Note that the present invention is not limited to the case where the main fixing portion 272 is located on the proximal side (the direction of the arrow A) with respect to the provisional fixing portion 271. The main fixing portion 272 may be located in a distal direction of the provisional fixing portion 271 (the direction of the arrow B). In addition, the provisional fixing portions 271 and the main fixing portion 272 are not limited to the case of being formed at different positions in one first hole 27A. For example, the provisional fixing portion 271 and the main fixing portion 272 may be provided in two respective holes separated from each other in the axial direction of the housing 12. In addition, the fixing portion 27 is not limited to the first hole 27A penetrating the circumferential wall of the housing 12. For example, the fixing portion 27 may be a recess provided in an inner circumferential surface of the circumferential wall of the housing 12 and recessed in a radial direction.

A pair of reception portions 28 are provided separate from each other in the circumferential direction of the housing 12. The pair of reception portions 28 constitute a reception portion row 281 (reception structure). The pair of reception portions 28 are located in the distal direction (the direction of the arrow B) with respect to the fixing portion 27. The pair of fixing portions 27 and the pair of reception portions 28 are arranged in a straight line shape along the axial direction of the housing 12. As illustrated in Fig. 7B, a pair of reception portion rows 281 are provided in the circumferential direction of the housing 12. In other words, the housing 12 includes two pairs of reception portion rows 281. The two pairs of reception portion rows 281 face each other with the central axis of the housing 12 interposed therebetween. The two pairs of reception portion rows 281 may not be arranged at positions facing each other with the central axis of the housing 12 interposed therebetween. Note that the present invention is not limited to the case where the two pairs of reception portion rows 281 are provided. The present invention is not limited thereto, as long as the number of the reception portions 28 is equal to the number of the fixing portions 27.

Each of the pair of reception portions 28 has a second hole 28A penetrating the circumferential wall of the housing 12. In the axial direction of the housing 12, each of a pair of second holes 28A and each of the end portions of the first region F1 of the fixing portion 27 in the circumferential direction are arranged in a straight line shape. Note that the reception portion 28 is not limited to the second hole 28A penetrating the circumferential wall of the housing 12. For example, the reception portion 28 may be a recess provided in the inner circumferential surface of the circumferential wall of the housing 12 and recessed in the radial direction.

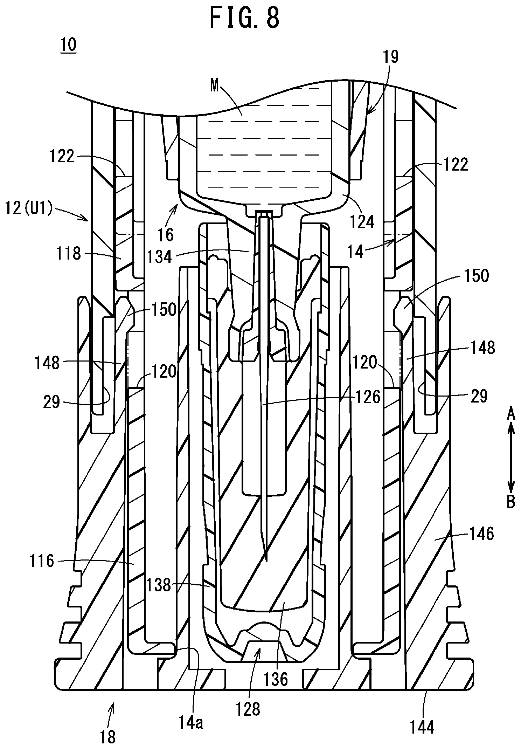

As illustrated in Fig. 8, a pair of recesses 29 are formed at the distal end of the housing 12. The recesses 29 have a rectangular cross section and are recessed in the radial direction with respect to the inner circumferential surface of the housing 12. The pair of recesses 29 are located at positions symmetric about an axis line of the housing 12, in an outer circumferential surface of the housing 12. When the cap 18 is mounted on the distal end of the housing 12, the recesses 29 are located at positions facing holding arms 148 of the cap 18.

As illustrated in Fig. 5, the needle cover 14 is movably located in the housing 12. As illustrated in Fig. 4, the needle cover 14 includes a cylindrical main structure portion 116, a pair of cover portions 118 extending from the main structure portion 116 toward the proximal side (the direction of the arrow A), and a pair of extension portions 119 extending from respective proximal ends of the cover portions 118.

The main structure portion 116 is located on a distal side of the needle cover 14 (the direction of the arrow B) and has, at a center of the main structure portion 116, a hole portion 14a that penetrates the main structure portion 116 in the axial direction and is open. The cover portions 118 are located symmetric about an axis line of the needle cover 14 and extend along the axial direction.

The main structure portion 116 includes a pair of engagement holes 120. The cover portions 118 include respective syringe guide holes 122 elongated along the axial direction. Each of the syringe guide holes 122 and each of the engagement holes 120 are arranged in a straight line shape along an axial direction of the needle cover 14. The syringe guide holes 122 and the engagement holes 120 are separated from each other by a predetermined distance in the axial direction of the needle cover 14.

The holding arms 148 of the cap 18 are engaged with the engagement holes 120. The engagement holes 120 radially penetrate the main structure portion 116. The engagement holes 120 have an oblong shape elongated in a direction orthogonal to the axial direction of the needle cover 14. The syringe guide holes 122 radially penetrate the cover portions 118. The syringe holder 19 is engaged with the syringe guide holes 122 in a movable manner in the axial direction. The syringe guide holes 122 have a guiding function of guiding the syringe holder 19 in the axial direction.

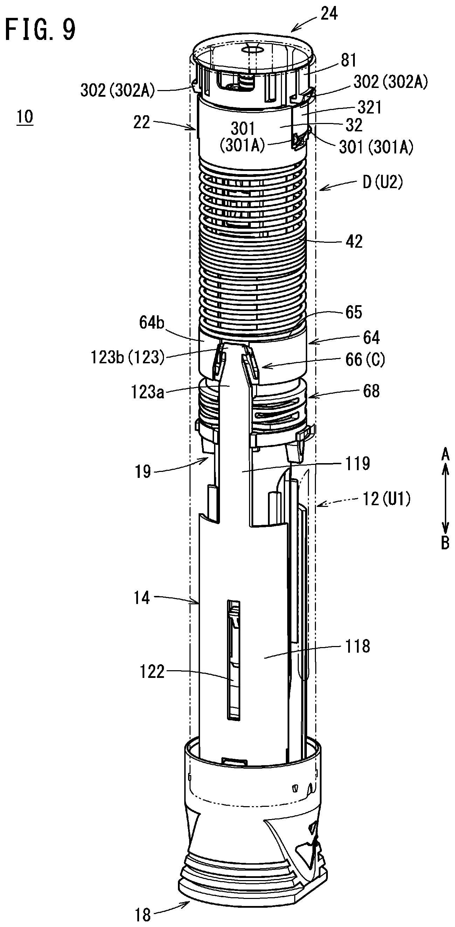

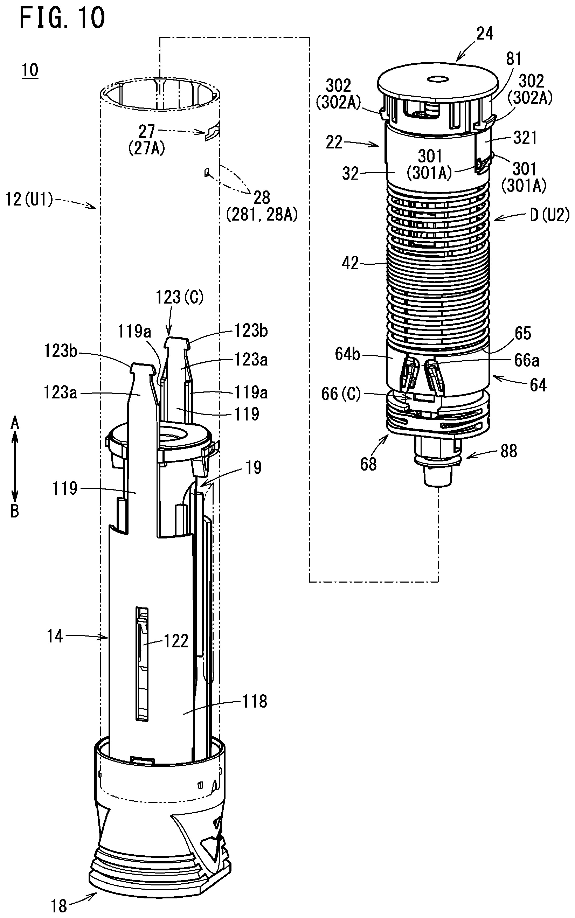

As illustrated in Fig. 9, the extension portions 119 further extend from centers of the proximal ends of the cover portions 118 toward the proximal end. The extension portion 119 extends having a substantially constant width in a straight line shape. A proximal end of each extension portion 119 includes a second connection portion 123 of a coupling mechanism C. As illustrated in Fig. 10, each extension portion 119 includes a pair of ribs 119a. The ribs 119a are provided at both ends of each extension portion 119 in a width direction thereof and extend along each extension portion 119. The ribs 119a project from an inner surface of each extension portion 119. A proximal end 119b of each rib 119a is located in the vicinity of a proximal end of each extension portion 119.

The second connection portions 123 are a part of the coupling mechanism C. The second connection portions 123 are capable of being coupled to first connection portions 66 of a sliding member 64, respectively. The coupling mechanism C has the first and second connection portions 66 and 123 and is capable of coupling a distal end of the sliding member 64 to a proximal end of the needle cover 14.

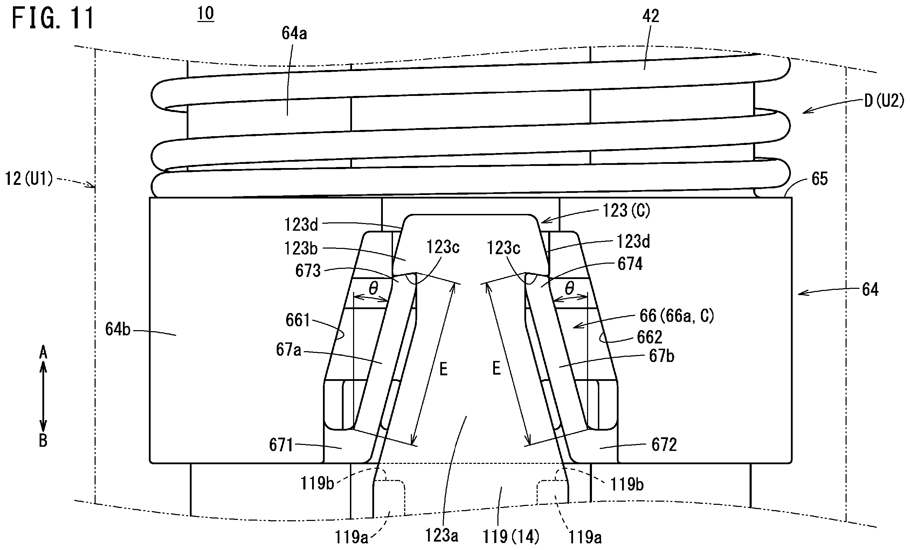

As illustrated in Fig. 11, the second connection portion 123 has an extension end 123a that gradually narrows with respect to each extension portion 119, a projection portion 123b located on a proximal end of the extension end 123a, and eave portions 123c located at a connection area between the extension end 123a and the projection portion 123b.

The extension end 123a is inclined to gradually narrow from the proximal end of each extension portion 119 toward a proximal direction (the direction of the arrow A). Side portions of the extension end 123a in a width direction thereof are inclined.

The side portions of the extension end 123a in the width direction and first and second flexible arm portions 67a and 67b are substantially parallel to each other. In other words, inclination angles of the side portions of the extension end 123a in the width direction are substantially equal to angles θ of the first and second flexible arm portions 67a and 67b.

The projection portion 123b projects to both sides in the width direction orthogonal to an extension direction of the extension end 123a and the extension portion 119. The projection portion 123b is wider than the extension end 123a in the width direction that is a circumferential direction of a cylindrical portion 64b. A maximum width dimension of the projection portion 123b is substantially equal to or smaller than a width dimension between the first flexible arm portion 67a and the second flexible arm portion 67b on the distal side. A maximum width dimension of the projection portion 123b is larger than a width dimension between the first flexible arm portion 67a and the second flexible arm portion 67b on the proximal side. Both ends of the projection portion 123b in the width direction have respective bevels 123d. The bevels 123d are inclined toward a center in the width direction of the projection portion 123b in a direction away from the extension end 123a (proximal direction).

The eave portion 123c is located at a connection area between a distal end of the projection portion 123b and a proximal end of the extension end 123a. The eave portions 123c are recessed inward in the width direction from respective end portions of the projection portion 123b in the width direction. The second connection portion 123 has substantially a T-shape including the extension end 123a and the projection portion 123b.

As illustrated in Fig. 1, the cap 18 is detachably attached to the distal end of the housing 12. As illustrated in Fig. 8, the cap 18 has a bottomed cylindrical shape including a bottom wall 144 formed at a distal end of the cap and an annular circumferential wall 146 erected from the bottom wall 144 toward the proximal side (the direction of the arrow A). A proximal end of the cap 18 is open. The cap 18 includes a pair of holding arms 148 projecting in the axial direction (the direction of the arrow A) from a proximal end of the circumferential wall 146.

The holding arms 148 are located radially inward with respect to the circumferential wall 146 and are radially tiltable with connection areas to the circumferential wall 146 as fulcrums, respectively. A proximal end of the holding arm 148 includes an outer hook 150 projecting radially inward. The pair of holding arms 148 are arranged at positions symmetric about an axis center of the cap 18.

When the cap 18 is mounted on the distal end of the housing 12, the proximal end of the circumferential wall 146 abuts the distal end of the housing 12, and the pair of holding arms 148 are inserted into a gap between the housing 12 and the needle cover 14. The pair of holding arms 148 are located at respective positions facing the recesses 29 and the engagement holes 120, and the outer hooks 150 are engaged with the engagement holes 120, respectively.

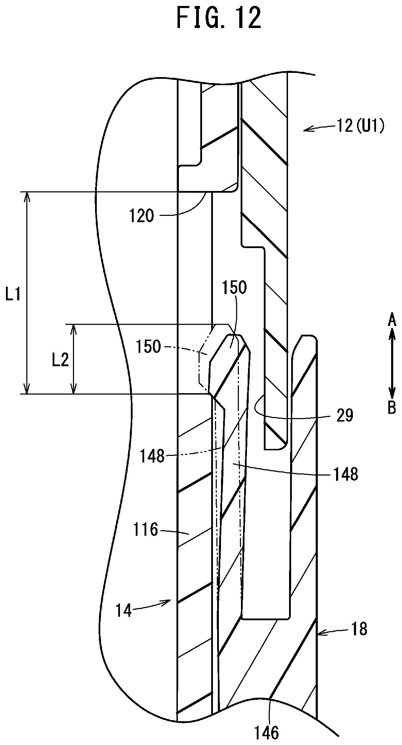

As illustrated in Fig. 12, the engagement holes 120 have an axial length L1 larger than an axial length L2 of the outer hooks 150 (L1 > L2). The outer hooks 150 are engaged with the engagement holes 120, respectively, in a movable manner in the axial direction (the directions of the arrows A and B).

As illustrated in Fig. 8, when the cap 18 is mounted on a distal end of the needle cover 14, the recesses 29 are located to be offset toward the distal side (the direction of the arrow B) with respect to the engagement holes 120. The proximal ends of the holding arms 148 having the outer hooks 150 are located on the proximal side (the direction of the arrow A) with respect to proximal ends of the recesses 29.

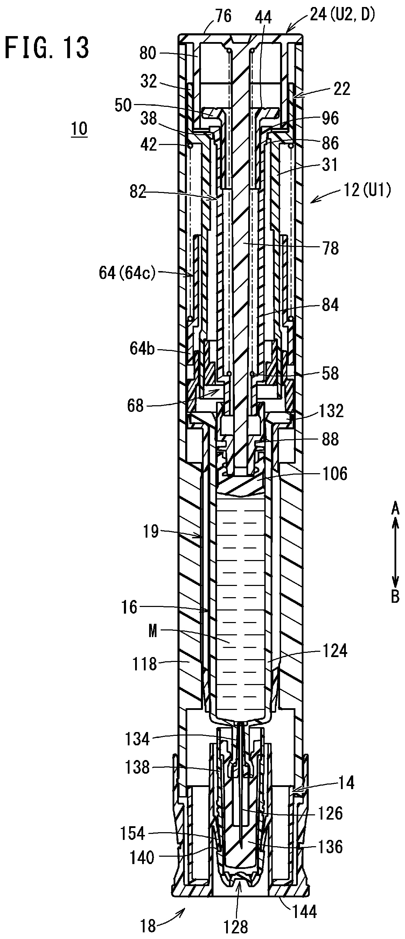

As illustrated in Fig. 13, an inner hook 154 projecting radially inward is located in the cap 18. An outer cover member 138 of a protective cover 128 is inserted into the inner hook 154. The inner hook 154 is engaged with an annular groove 140 of the outer cover member 138. Accordingly, the cap 18 is engaged with the protective cover 128 via the inner hook 154 and is engaged with the distal end of the needle cover 14. In a state where the cap 18 holds the protective cover 128, the cap 18 is mounted to cover the distal ends of the needle cover 14 and the housing 12.

As illustrated in Fig. 5, the syringe holder 19 is formed in a cylindrical shape and is housed in the housing 12. The syringe holder 19 includes a pair of guide portions 142 projecting radially outward from an outer circumferential surface of the syringe holder. The guide portions 142 are inserted into the syringe guide holes 122 of the needle cover 14, respectively. Accordingly, the syringe holder 19 is held inside the needle cover 14, together with the syringe 16, in a movable manner along the axial direction (the directions of the arrows A and B).

When the syringe holder 19 holding the syringe 16 is housed in the needle cover 14, a puncture needle 126 is located on the distal side (the direction of the arrow B), and the barrel 124 faces the viewing window 26 (see Fig. 1) that is open in the housing 12.

As illustrated in Fig. 3, the second unit U2 is attached to the fixing portion 27 of the housing 12 in the first unit U1. At least a part of the second unit U2 is housed in the housing 12 in a direction from the proximal end toward the distal end of the housing 12. The second unit U2 includes a drive unit D that biases a gasket 106 of the syringe 16 toward the distal end of the housing 12.

As illustrated in Fig. 2, the second unit U2 includes a first fixing engagement portion 301 for performing provisional fixing to the first unit U1 and a second engagement portion 302 for performing main fixing to the first unit U1.

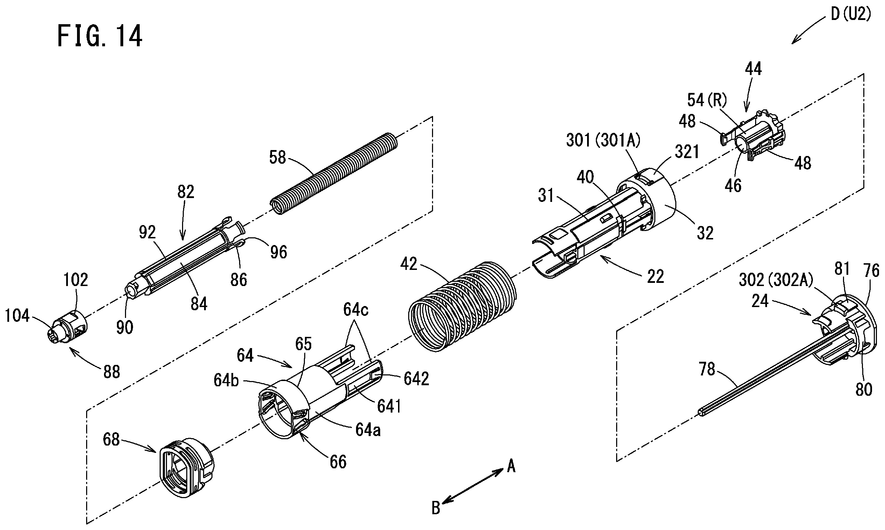

As illustrated in Fig. 14, the drive unit D is configured by assembling a sleeve body 22, an end cap 24, an injection spring 58, a plunger 82, a top member 88, an end guide 68, a sleeve spring 42, a locking pin 44, and the sliding member 64.

The end cap 24 is located at a proximal end of the drive unit D, and the end guide 68 and the top member 88 are located at a distal end of the drive unit D (see Fig. 10). The first connection portions 66 of the sliding member 64 are located adjacent to the end guide 68.

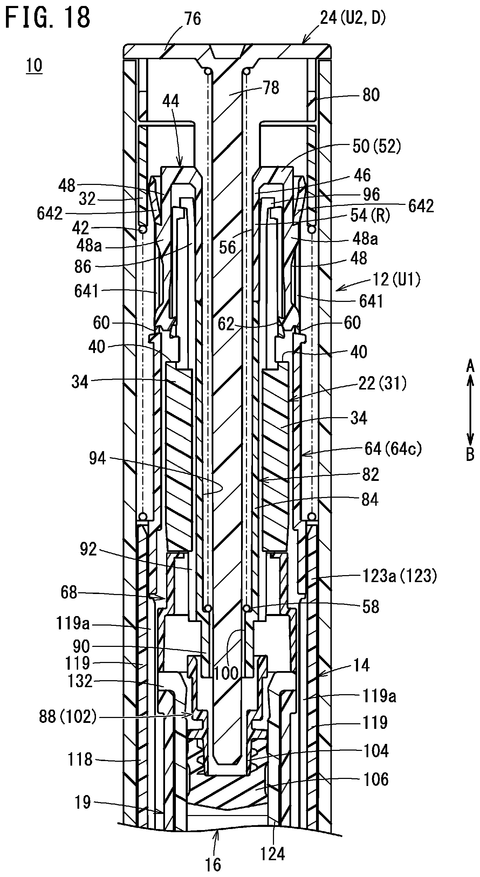

When the liquid medicine is administered by the liquid medicine administration device 10, in an initial state where the puncture needle 126 is covered with the needle cover 14, the drive unit D has a locking mechanism R (see Fig. 18) that inhibits the plunger 82 from moving in the distal direction which is caused by the injection spring 58. When the needle cover 14 is pressed and moved in the proximal direction, a locked state by the locking mechanism R is unlocked, thereby allowing the plunger 82 to move in the distal direction.

The sleeve body 22 is housed on the proximal side (the direction of the arrow A) of the housing 12. The sleeve body 22 is formed in a hollow shape. The sleeve body 22 has a body main structure 31 formed in a cylindrical shape and an enlarged diameter portion 32 formed on a proximal side (the direction of the arrow A) of the body main structure 31. The body main structure 31 has a substantially constant diameter along the axial direction (the directions of the arrows A and B). The body main structure 31 includes four guide ribs 34 projecting radially inward from an inner circumferential surface of the body main structure.

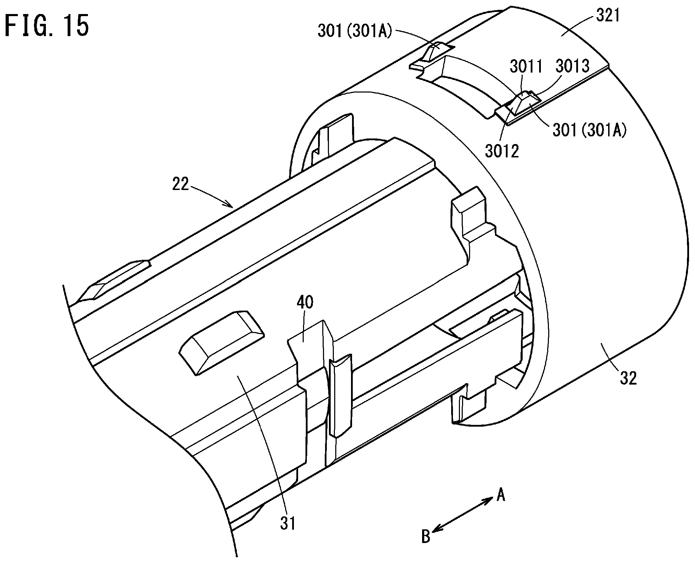

As illustrated in Fig. 15, the enlarged diameter portion 32 is formed in a cylindrical shape. The enlarged diameter portion 32 has, on the outer circumferential surface thereof, a pair of projecting portions 321 projecting radially outward. The pair of projecting portions 321 are located at positions separated from each other by 180° in a circumferential direction of the sleeve body 22. When viewed from an outer circumferential side of the enlarged diameter portion 32, each projecting portion 321 has a substantially rectangular shape and extends in a direction from the proximal end toward a distal end of the sleeve body 22. A distal portion of each projecting portion 321 has a pair of first engagement portions 301.

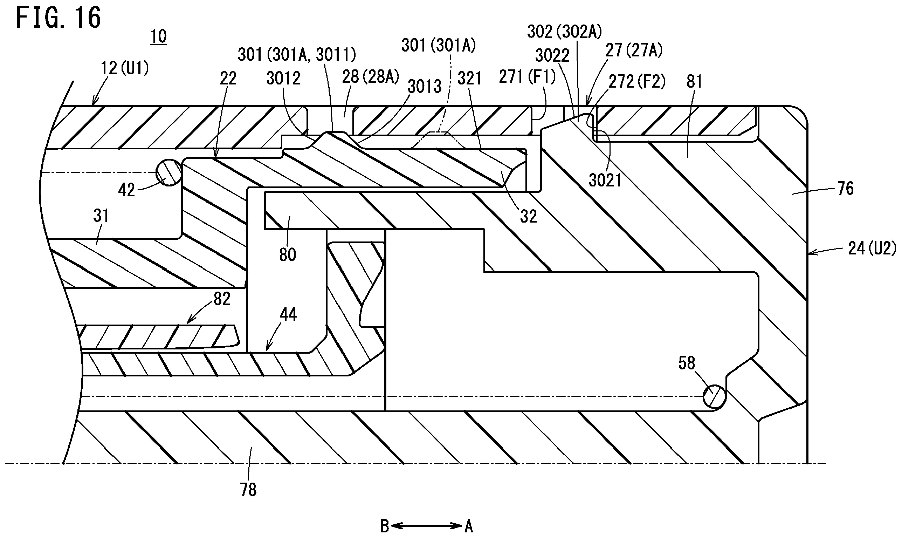

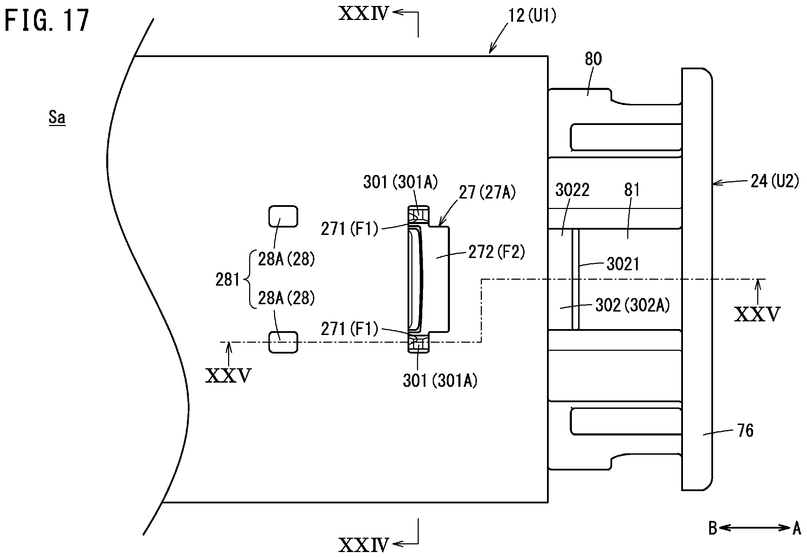

The pair of first engagement portions 301 are separated from each other in the circumferential direction of the sleeve body 22. The pair of first engagement portions 301 are provided as a set to be separated from each other in the circumferential direction of the housing 12. In other words, the housing 12 includes two pairs of first engagement portions 301. Each of the pair of first engagement portions 301 has a first engagement protrusion 301A projecting radially outward from an outer circumferential surface of the projecting portion 321. The first engagement protrusions 301A are separated from each other in the circumferential direction of the sleeve body 22. As illustrated in Fig. 16, the first engagement protrusions 301A are each formed in a substantially trapezoidal shape tapered in a direction away from the outer circumferential surface of the projecting portion 321. An apex portion 3011 of the first engagement protrusion 301A is substantially parallel to an axial direction of the sleeve body 22. The first engagement protrusion 301A has a distal inclined portion 3012 located in the distal direction (the direction of the arrow B) with respect to the apex portion 3011 and a proximal inclined portion 3013 located in the proximal direction (the direction of the arrow A) with respect to the apex portion 3011. The distal inclined portion 3012 is inclined from the apex portion 3011 toward the outer circumferential surface of the projecting portion 321. The proximal inclined portion 3013 is inclined from the apex portion 3011 toward the outer circumferential surface of the projecting portion 321. As illustrated in Fig. 3, when the intermediate assembly Sa is formed by performing provisional fixing of the first unit U1 and the second unit U2, the pair of first engagement portions 301 are engaged with the pair of provisional fixing portions 271 of the fixing portion 27, respectively (see Fig. 17). The number of the first engagement portions 301 is equal to the number of the fixing portions 27.

As illustrated in Fig. 5, the guide ribs 34 are individually separated from each other at 90-degree intervals along the circumferential direction of the body main structure 31. The number of guide ribs 34 is not limited to four. At least three guide ribs 34 are provided. The guide rib 34 has a substantially constant thickness and projects radially inward in a straight line shape from the inner circumferential surface of the body main structure. The guide ribs 34 have the same radially projecting length. A set of guide ribs 34 is located to face each other with an axis center of the body main structure 31 interposed therebetween.

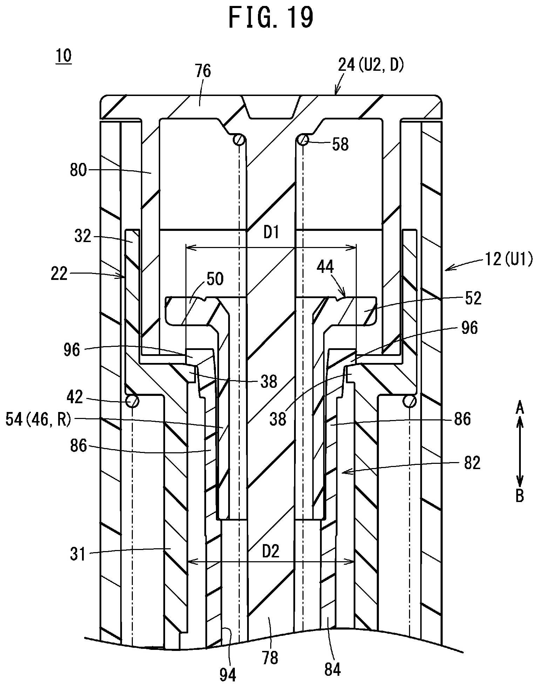

As illustrated in Fig. 18, the sleeve body 22 houses the locking pin 44 and the plunger 82 to be described below in a movable manner in the axial direction. As illustrated in Fig. 19, a plurality of protrusion portions 38 projecting radially inward are provided at a boundary portion between the enlarged diameter portion 32 and the body main structure 31. The protrusion portions 38 have a rectangular cross section and project radially outward from the inner circumferential surface of the body main structure 31. Proximal ends of the protrusion portions 38 are bevels that are gradually inclined inward in the radial direction.

The protrusion portions 38 are engaged with flanges 96 of flexible portions 86 in the plunger 82 to be described below on the proximal side (the direction of the arrow A). The number and arrangement of the protrusion portions 38 correspond to the number and arrangement of the flexible portions 86.

The sleeve body 22 is housed in the housing 12 from the proximal end of the housing 12, and the enlarged diameter portion 32 is engaged with an inner circumferential area of the housing 12. Accordingly, a proximal end of the enlarged diameter portion 32 is housed and fixed at a position on the distal side (the direction of the arrow B) by a predetermined distance away from the proximal end of the housing 12.

As illustrated in Fig. 18, an outer circumferential surface of the body main structure 31 has a pair of locking grooves 40 that are recessed radially inward. The pair of locking grooves 40 are located at positions symmetric about an axis center of the sleeve body 22, and locking claws 62 of the locking pin 44 to be described below are engaged with the locking grooves 40.

A sleeve spring 42 is located between the outer circumferential surface of the body main structure 31 and the inner circumferential surface of the housing 12. The sleeve spring 42 is a spring member including a coil spring. A proximal end of the sleeve spring 42 is engaged with a distal end of the enlarged diameter portion 32. A distal end of the sleeve spring 42 is engaged with the sliding member 64 to be described below. A spring force of the sleeve spring 42 biases the sliding member 64 in a direction away from the sleeve body 22, that is, in the distal direction (direction of the arrow B).

The sleeve body 22 houses the locking pin 44 that straddles the enlarged diameter portion 32 and the body main structure 31. The locking pin 44 is movable in the axial direction (the directions of the arrows A and B) inside the sleeve body 22. The locking pin 44 has a cylindrical pin main structure 46 formed at a center thereof and a pair of arm portions 48 provided on an outer circumferential side of the pin main structure 46. The arm portions 48 are connected to a proximal end of the pin main structure 46 in an integrated manner.

The pin main structure 46 includes a flat end wall portion 50 that is formed at a proximal end thereof and is orthogonal to the axial direction, a large diameter portion 52 extending from a center of the end wall portion 50 toward the distal side (the direction of the arrow B), and a small diameter portion 54 that is formed on a distal side (the direction of the arrow B) of the large diameter portion 52 and has a diameter smaller than that of the large diameter portion 52. The pin main structure 46 has a hole portion 56 at an axis center thereof. The hole portion 56 extends along the axial direction with a constant diameter and penetrates, in a straight line shape, the end wall portion 50, the large diameter portion 52, and the small diameter portion 54. A shaft 78 of the end cap 24 and an injection spring 58 to be described below are inserted into the hole portion 56.

Proximal ends of the arm portions 48 are connected to the end wall portion 50 of the pin main structure 46 and are arranged in parallel at predetermined intervals on the outer circumferential side of the pin main structure 46. The arm portions 48 extend individually toward the distal end (direction of the arrow B) to have the same length. A distal end of each arm portion 48 has an engagement end 60 that is engaged with the sliding member 64 to be described below, and the locking claws 62 formed on a radially inner side of the engagement end 60.

The engagement end 60 has a flat shape orthogonal to an extension direction of the arm portion 48. The locking claw 62 projects radially inward from the engagement end 60 and toward the distal side (the direction of the arrow B). The locking claw 62 has a triangular cross section tapered toward the distal end of the arm portion 48.

A protruding portion 48a projecting outward is provided at a center of the arm portion 48 in a longitudinal direction thereof. The protruding portion 48a projects outward from an outer surface of the arm portion 48 and has an oblong shape elongated in a direction orthogonal to the extension direction of the arm portion 48. The protruding portion 48a is inserted into a slit hole 641 of the sliding member 64 to be described below.

Inside the housing 12, the cylindrical sliding member 64 is provided on the distal side (the direction of the arrow B) of the locking pin 44.

The sliding member 64 is movable in the axial direction (the directions of the arrows A and B) inside the housing 12. The plunger 82 and the body main structure 31 of the sleeve body 22 are inserted into the sliding member 64.

As illustrated in Fig. 14, the sliding member 64 has a cylindrical sleeve main structure 64a, the cylindrical portion 64b located at a distal end of the sleeve main structure 64a, and a pair of engagement arms 64c extending from a proximal end of the sleeve main structure 64a. The cylindrical portion 64b is more enlarged in diameter than a diameter of the sleeve main structure 64a and is larger in diameter than that of the sleeve main structure 64a. A boundary portion between the sleeve main structure 64a and the cylindrical portion 64b has a receiving portion 65 formed in a step shape. As illustrated in Fig. 9, the distal end of the sleeve spring 42 located on an outer circumference of the sliding member 64 is held in the receiving portion 65. The spring force of the sleeve spring 42 biases the sliding member 64 toward the distal end.

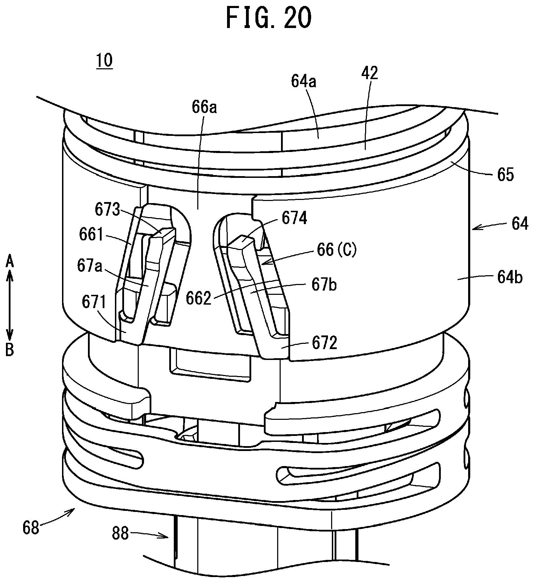

As illustrated in Fig. 20, the cylindrical portion 64b of the sliding member 64 has the first connection portions 66. The first connection portions 66 are a part of the coupling mechanism C. The first connection portions 66 are provided in a pair to be separated from each other along a circumferential direction of the cylindrical portion 64b. The first connection portions 66 are symmetric about an axis line of the sliding member 64. Each first connection portion 66 has a housing recess 66a and first and second flexible arm portions 67a and 67b provided in the housing recess 66a. The housing recess 66a is recessed radially inward from an outer circumferential surface of the cylindrical portion 64b and is open from a distal end toward a proximal end of the cylindrical portion 64b. As illustrated in Fig. 11, the housing recess 66a has a trapezoidal shape which narrows toward a proximal end of the sliding member 64. In other words, side walls 661 and 662 constituting both sides of the housing recess 66a in the circumferential direction of the cylindrical portion 64b are inclined inward from respective distal ends toward respective proximal ends of the side walls.

The first and second flexible arm portions 67a and 67b are separated from each other along the circumferential direction of the cylindrical portion 64b and are respectively arranged along the inclined side walls 661 and 662 of the housing recess 66a. The first flexible arm portion 67a and the second flexible arm portion 67b are symmetrical about a center of the housing recess 66a. The first and second flexible arm portions 67a and 67b are separated from the side walls 661 and 662 of the housing recess 66a and are in parallel to the side walls 661 and 662.

Ends on one side (end portions in the direction of the arrow B) of the first and second flexible arm portions 67a and 67b are fixed ends 671 and 672 and are connected to the distal end of the cylindrical portion 64b. The first and second flexible arm portions 67a and 67b are individually inclined at an angle θ, with the fixed ends 671 and 672 of the first and second flexible arm portions 67a and 67b as base points. The angle θ is, for example, within a range of 5° to 45°. Ends on the other side (end portions in the direction of the arrow A) of the first and second flexible arm portions 67a and 67b are free ends 673 and 674 and are not connected to the cylindrical portion 64b. The fixed ends 671 and 672 of the first and second flexible arm portions 67a and 67b are closer to the distal end of the cylindrical portion 64b than the free ends 673 and 674.

The first and second flexible arm portions 67a and 67b have a tiltable cantilever structure with the fixed ends 671 and 672 as the base points. The first and second flexible arm portions 67a and 67b are elastically deformable. A length E from the fixed ends 671 and 672 to the free ends 673 and 674 of the first and second flexible arm portions 67a and 67b is, for example, 3 mm or more along an extension direction of the first and second flexible arm portions 67a and 67b. The first flexible arm portion 67a and the second flexible arm portion 67b have the same length.

A space is formed between the first flexible arm portion 67a and the second flexible arm portion 67b in the housing recess 66a, and the second connection portion 123 of the needle cover 14 which is a part of the coupling mechanism C is housed in the space.

As illustrated in Fig. 14, in the sliding member 64, the pair of engagement arms 64c are symmetric about the axis line of the sliding member 64, and each engagement arm 64c projects in a proximal direction of the sliding member 64. The engagement arm 64c has the slit hole 641. The slit hole 641 has an oblong shape along an extension direction of the engagement arms 64c. A proximal end of the slit hole 641 has a projecting piece 642 projecting from a proximal end toward a distal end of the engagement arm 64c. The projecting piece 642 is inclined inward toward a distal end thereof.

As illustrated in Fig. 18, a part of the arm portion 48 of the locking pin 44 is inserted into the slit hole 641. The engagement end 60 of the locking pin 44 is engageable with the distal end of the slit hole 641. The protruding portions 48a of the arm portions 48 are engageable with the projecting pieces 642, respectively. Accordingly, the locking pin 44 is engaged with the sliding member 64, and the locking pin 44 is prevented from being detached from the sliding member 64 in the proximal direction.

As illustrated in Fig. 9, the end guide 68 is located to face the distal end of the sliding member 64. The end guide 68 has a cylindrical shape having a diameter substantially equal to that of the sliding member 64. As illustrated in Fig. 18, the plunger 82 is inserted into the end guide 68 in a movable manner in the axial direction.

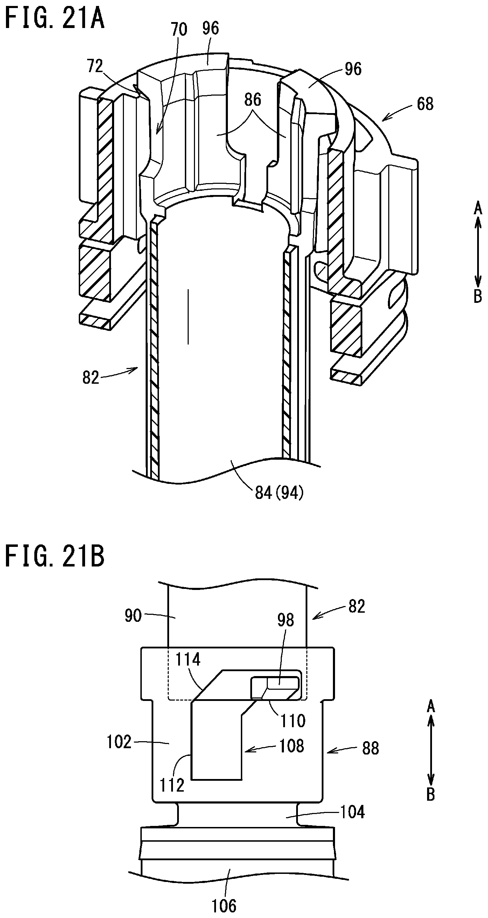

As illustrated in Fig. 21A, an inner circumferential surface of the end guide 68 has guide grooves 70 for rotating the plunger 82. The guide grooves 70 are recessed radially outward with respect to the inner circumferential surface of the end guide 68. The flanges 96 of the flexible portions 86 in the plunger 82 to be described below are inserted into the guide grooves 70. The guide grooves 70 are arranged at four locations at equal intervals in the circumferential direction to correspond to the number and arrangement of the flexible portions 86.

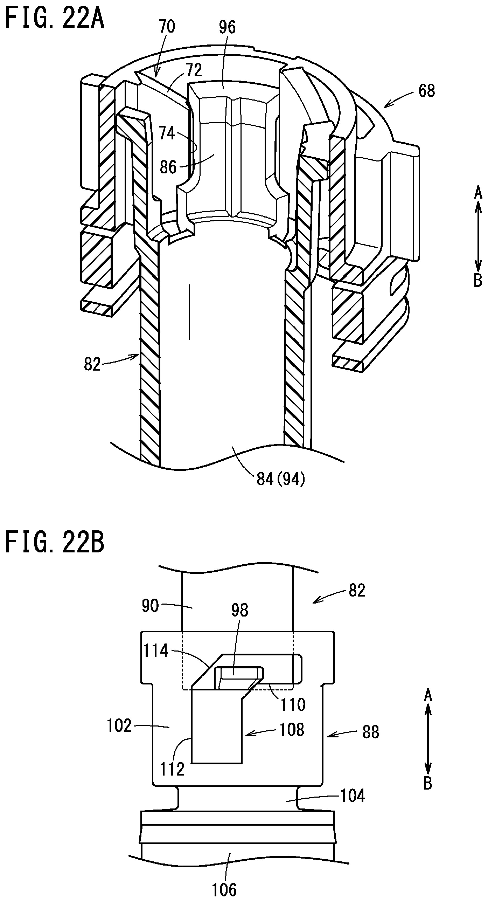

As illustrated in Fig. 22A, each guide groove 70 includes an inclined guide portion 72 and a straight guide portion 74. The inclined guide portion 72 is inclined in the distal direction (the direction of the arrow B) with respect to an axial direction (the directions of the arrows A and B) of the end guide 68. The straight guide portion 74 extends in a straight line shape from a distal end of the inclined guide portion 72. A width dimension along a circumferential direction of the straight guide portion 74 is substantially equal to or slightly larger than a width dimension of the flange 96 in the plunger 82 to be described below.

As illustrated in Fig. 18, the end cap 24 has a lid 76 that closes the proximal end of the housing 12, and the shaft 78 extending from a center of the lid 76 toward the distal side (the direction of the arrow B). The lid 76 has a disk shape having a diameter equal to an outer diameter of the housing 12.

The lid 76 has a pusher 80 projecting from an end surface of the lid on the distal side, and a pair of connection pieces 81 projecting from a distal end of the pusher 80. The pusher 80 is formed in an annular shape projecting from the end surface of the lid 76. When the pusher 80 abuts the proximal end of the enlarged diameter portion 32 of the sleeve body 22, the sleeve body 22 housed in the housing 12 is restricted from moving in the proximal direction (the direction of the arrow A) and held.

As illustrated in Fig. 4, the pusher 80 has a pair of second engagement portions 302. The pair of second engagement portions 302 are provided separately from each other along a circumferential direction of the end cap 24. Each of the pair of second engagement portions 302 has a second engagement protrusion 302A projecting radially outward from an outer circumferential surface of the pusher 80. The second engagement portion 302 includes a latching portion 3021 provided at a proximal portion of the second engagement portion and an inclined area 3022 extending from the latching portion 3021 to a distal side thereof. The latching portion 3021 is orthogonal to an axial direction of the needle hub 24. The inclined area 3022 is inclined radially inward from an outer end of the latching portion 3021 in a radial direction thereof toward the distal direction (the direction of the arrow B). As illustrated in Fig. 1, when main fixing of the first unit U1 and the second unit U2 are performed, the second engagement portion 302 is engaged with the main fixing portion 272 of the fixing portion 27 (see Fig. 16). As illustrated in Fig. 6, the second engagement protrusion 302A is engaged with the main fixing portion 272 of the first hole 27A in the housing 12. Specifically, when the latching portion 3021 is engaged with the main fixing portion 272 on a proximal end side thereof, the end cap 24 is prevented from moving in the proximal direction (the direction of the arrow A) with respect to the housing 12. When the end cap 24 is fixed to the housing 12, the end cap 24 is positioned in the axial direction and in the circumferential direction with respect to the housing 12.

Note that the present invention is not limited to the case where the first engagement portion 301 is provided on the sleeve body 22, and the second engagement portion 302 is provided on the end cap 24. The first engagement portion 301 may be provided on the end cap 24, and the second engagement portion 302 may be provided on the sleeve body 22.

As illustrated in Fig. 5, the shaft 78 is a shaft structure extending toward the distal side (the direction of the arrow B) along the axial direction. The shaft 78 is housed in the housing 12 and the sleeve body 22 and extends to the vicinity of a center of the housing 12 in the axial direction. The insertion of the injection spring 58 and the plunger 82 is performed on an outer circumferential side of the shaft 78. The injection spring 58 is a coil spring and is elongated corresponding to a length of the shaft 78 in the axial direction. The injection spring 58 is interposed between the plunger 82 to be described below and the end surface of the lid 76, and a spring force of the injection spring 58 biases the plunger 82 in the distal direction (the direction of the arrow B).

When the end cap 24 is mounted on the proximal end of the housing 12, the open proximal end is closed by the lid 76, and the shaft 78 is located on the axis line of the housing 12.

As illustrated in Fig. 18, the plunger 82 has a cylindrical shape elongated in the axial direction (the directions of the arrows A and B). The plunger 82 includes a rod main structure 84 having a constant diameter along the axial direction, a plurality of flexible portions 86 formed on a proximal side (the direction of the arrow A) of the rod main structure 84 and divided in the circumferential direction, and a mounting portion 90 which is formed on a distal side (the direction of the arrow B) of the rod main structure 84 and on which the top member 88 to be described below is mounted.

The rod main structure 84 has a circular cross section. As illustrated in Fig. 4, four sliding grooves 92 that are recessed radially inward are formed in an outer circumferential surface of the rod main structure 84. The sliding grooves 92 have a rectangular cross section and are recessed with respect to the outer circumferential surface of the rod main structure 84. As illustrated in Fig. 18, the guide ribs 34 of the sleeve body 22 are inserted into the sliding grooves 92. The sliding grooves 92 are separated at equal intervals in the circumferential direction in corresponding to the number and arrangement of the guide ribs 34. The four sliding grooves 92 correspond to the guide ribs 34.

When the plunger 82 is housed in the sleeve body 22, distal ends of the guide ribs 34 are inserted into the sliding grooves 92, respectively.

A first rod hole 94 extending along the axial direction (the directions of the arrows A and B) is provided in the rod main structure 84, and the shaft 78 of the end cap 24 and the injection spring 58 are inserted into the first rod hole 94.

The flexible portions 86 project in the axial direction (the direction of the arrow A) from a proximal end of the rod main structure 84 and are located separately from each other in the circumferential direction. The flexible portion 86 is located between two adjacent sliding grooves 92. A proximal side (the direction of the arrow A) of the flexible portions 86 is radially tiltable with a distal side (the direction of the arrow B) of the flexible portions 86 connected to the rod main structure 84 as a fulcrum. As illustrated in Fig. 19, the flexible portion 86 includes, at the proximal end thereof, the flange 96 flaring radially outward. Surfaces of the flanges 96 on the distal side (the direction of the arrow B) are bevels that are inclined radially outward and gradually toward the proximal side and.

A diameter of the flexible portions 86 is enlarged radially outward with respect to the rod main structure 84. The small diameter portion 54 of the locking pin 44 is inserted into the flexible portions 86. An inner circumference diameter of the flexible portions 86 is larger than an inner circumference diameter of the first rod hole 94.

The flanges 96 of the flexible portions 86 have an outer diameter D1 slightly larger than an inner diameter D2 of the body main structure 31 of the sleeve body 22 (D1 > D2).

As illustrated in Fig. 14, the mounting portion 90 has a small diameter with respect to the rod main structure 84. An outer circumferential surface of the mounting portion 90 has a pair of engagement pieces 98 projecting radially outward. The pair of engagement pieces 98 are engaged with the top member 88 to be described below. The engagement pieces 98 extend in a horizontal direction orthogonal to the axial direction of the plunger 82 and are located at positions symmetric about an axis center of the plunger 82.

As illustrated in Fig. 18, a second rod hole 100 extending along the axial direction (the directions of the arrows A and B) is provided in the mounting portion 90. The second rod hole 100 is smaller in diameter than the first rod hole 94, and the shaft 78 of the end cap 24 is inserted into the second rod hole 100. A distal end of the injection spring 58 is engaged with a step portion formed at a boundary between the second rod hole 100 and the first rod hole 94. Accordingly, the spring force of the injection spring 58 biases the plunger 82 in a direction away from the end cap 24, that is, in the distal direction (the direction of the arrow B).

The top member 88 has a cup shape which is open on the proximal side (the direction of the arrow A). The top member 88 is rotatable relatively with respect to the mounting portion 90 of the plunger 82. The top member 88 includes a cylindrical cup portion 102 located on the proximal side, and an attachment portion 104 projecting from a distal end of the cup portion 102. A gasket 106 made of an elastic material is mounted on the top member 88 to cover an outer circumferential side of the attachment portion 104.

The mounting portion 90 of the plunger 82 is inserted into the cup portion 102 from the proximal side. As illustrated in Fig. 21B, a pair of guide grooves 108 which radially penetrate the cup portion and are inserted into the engagement pieces 98 of the plunger 82 are formed in an outer circumferential surface of the cup portion 102.

The guide grooves 108 are arranged in a pair to be symmetric about an axis center of the cup portion 102. The guide groove 108 has a horizontal portion 110 which is formed in the vicinity of a proximal end of the cup portion 102 and extends along the circumferential direction, and a perpendicular portion 112 which is orthogonal to the horizontal portion 110 and extends from one end in the circumferential direction toward the distal side (the direction of the arrow B). The guide groove 108 substantially has an L-shape including the horizontal portion 110 and the perpendicular portion 112. A connection area between the horizontal portion 110 and the perpendicular portion 112 has an inclined portion 114 inclined at a predetermined angle.

In the top member 88, the mounting portion 90 of the plunger 82 is inserted into the cup portion 102, and the engagement pieces 98 are inserted into the pair of guide grooves 108. Accordingly, the top member 88 and the plunger 82 are connected in the axial direction and are movable in an integrated manner along the axial direction. After the gasket 106 is mounted on the attachment portion 104 of the top member 88, the gasket 106 is inserted into the barrel 124 of the syringe 16 to be described below.

As illustrated in Fig. 5, the syringe 16 is housed in the needle cover 14 in the first unit U1. The syringe 16 includes the hollow barrel 124, the gasket 106 slidably inserted into the barrel 124, the puncture needle 126 that is provided at a distal end of the barrel 124 and projects in the distal direction (direction of the arrow B), and the protective cover 128 attached to the distal end of the barrel 124. As illustrated in Fig. 2, the syringe 16 is a unit in which the barrel 124, the gasket 106 (see Fig. 4), the puncture needle 126, and the protective cover 128 are integrally assembled.

As illustrated in Fig. 5, the cylindrical syringe holder 19 is located on an outer circumference of the barrel 124, and the barrel 124 is held by the syringe holder 19. Note that examples of the liquid medicine M to be used include those used for subcutaneous injection to a patient.

The barrel 124 is a hollow structure which is formed in a substantially cylindrical shape and has an opening portion at a proximal end thereof. The barrel 124 is filled with the liquid medicine M. A flange 132 projecting radially outward is provided on an outer circumferential portion of a proximal end of the barrel 124. A needle holding portion 134 is provided at the distal end of the barrel 124. The needle holding portion 134 has a diameter decreasing with respect to the barrel 124 and projects in the distal direction. The needle holding portion 134 holds a proximal end of the puncture needle 126.

The barrel 124 is made of a transparent resin material, and it is possible to visually check, from the outside through the viewing window 26 of the housing 12, a remaining amount of the liquid medicine M with which the barrel 124 is filled. A flange 132 of the outer cylinder 124 is engaged with the proximal end of the syringe holder 19. Accordingly, the syringe 16 does not move relative to the syringe holder 19 in the axial direction, and an outer circumferential side of the syringe 16 is covered and held by the syringe holder 19 in an integrated manner.

The gasket 106 is made of an elastic material such as rubber. The gasket 106 is mounted on the attachment portion 104 of the top member 88, and the gasket 106 is inserted into the barrel 124 via the opening portion at the proximal end of the barrel 124. The gasket 106 is slidably housed in the axial direction along an inner circumferential surface of the barrel 124. When the gasket 106 is inserted into the barrel 124, the proximal side of the barrel 124 is sealed in a liquid-tight manner, and the liquid medicine M is encased in the barrel 124.

The puncture needle 126 is a hollow body having a flow channel in which the liquid medicine M flows. The puncture needle 126 projects from the needle holding portion 134 in the distal direction. The flow channel of the puncture needle 126 communicates with the inside of the barrel 124 filled with the liquid medicine M. The liquid medicine M filling the barrel 124 is ejected from a distal end of the puncture needle 126 and administered to a patient.

The protective cover 128 is mounted on the distal end of the barrel 124 and covers the puncture needle 126. The protective cover 128 includes a needle shield 136 that is made of an elastic material such as rubber and is mounted on the needle holding portion 134, and an outer cover member 138 that covers an outer circumferential side of the needle shield 136. A proximal end of the needle shield 136 is mounted on the needle holding portion 134 to cover the puncture needle 126. The outer cover member 138 is slidably fitted into an outer circumferential surface of the needle shield 136. As illustrated in Fig. 13, an annular groove 140 recessed radially inward is formed in an outer circumferential surface of the outer cover member 138. The inner hook 154 of the cap 18 to be described below is engaged with the annular groove 140.

Next, a case will be described, in which the intermediate assembly Sa in which the second unit U2 is provisionally fixed to the first unit U1 is assembled in a state where the syringe 16 is not housed. Since a higher level of cleanliness is required for a manufacturing environment of the syringe 16 filled with the liquid medicine M than for a manufacturing environment of the first and second units U1 and U2, the intermediate assembly Sa is manufactured by assembling the first and second units U1 and U2 which require substantially the same level of the manufacturing environment before final assembling.

The intermediate assembly Sa is assembled as follows.

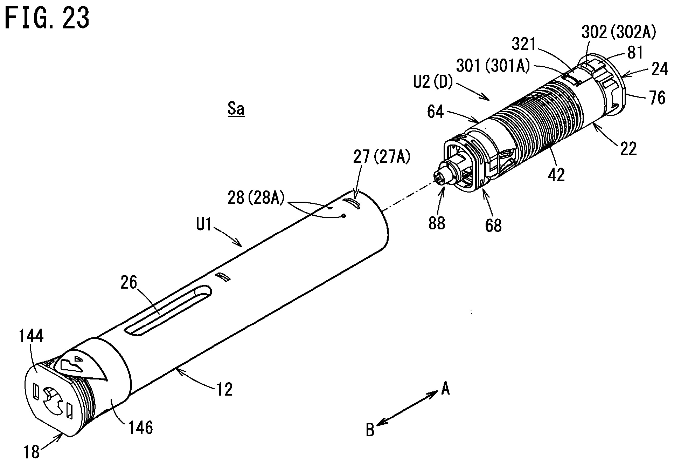

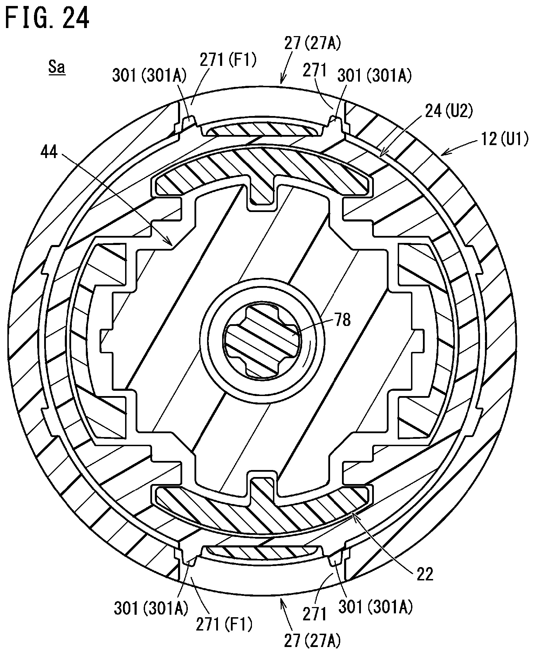

After a distal end of the second unit U2 is inserted into the proximal end of the housing 12 in the first unit U1 from the state illustrated in Fig. 23, the second unit U2 is moved in the distal direction (the direction of the arrow B) with respect to the first unit U1. The first engagement portion 301 of the second unit U2 is inserted into the housing 12. In this instance, the distal inclined portions 3012 of the first engagement portions 301 come into contact with the proximal end of the housing 12, so that the first engagement protrusions 301A move in the distal direction while pushing and expanding the inner circumferential surface of the housing 12 radially outward. As illustrated in Fig. 24, the pair of first engagement protrusions 301A are inserted into and engaged with the pair of provisional fixing portions 271 of the fixing portion 27 of the first unit U1 (see Fig. 17). Accordingly, the first unit U1 and the second unit U2 are brought into a provisional fixing state where relative movement in the axial direction and relative movement in the circumferential direction are restricted. The intermediate assembly Sa in which the first unit U1 and the second unit U2 are provisionally fixed is fully manufactured.

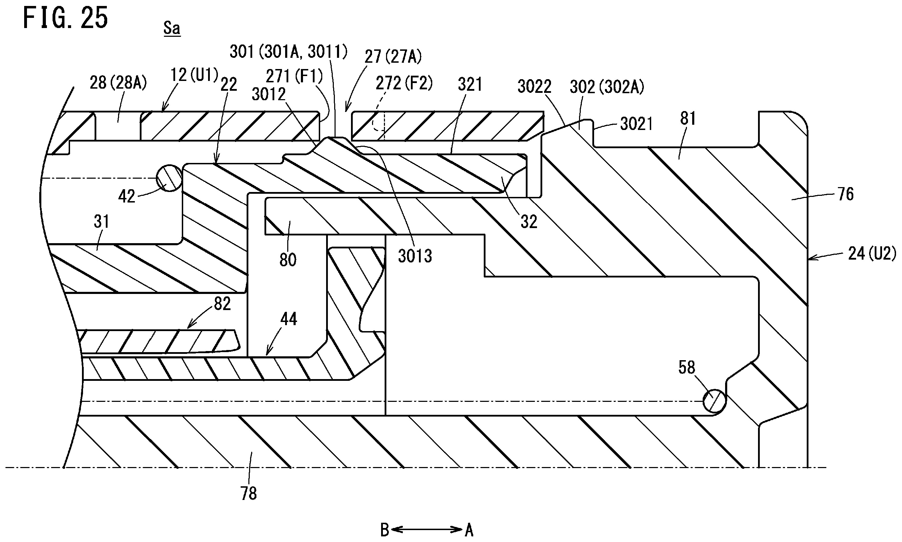

As illustrated in Fig. 3, the intermediate assembly Sa is in a provisionally assembled state where a part of the second unit U2 is housed in the first unit U1 in a state where the syringe 16 is not housed in the first unit U1, and a part of the second unit U2 projects in the proximal direction (the direction of the arrow A) with respect to the proximal end of the housing 12. Specifically, the lid 76 of the end cap 24 is separated from the proximal end of the housing 12 in the proximal direction (the direction of the arrow A), and a part of the pusher 80 is exposed to the outside. In this instance, as illustrated in Fig. 25, the second engagement portion 302 is located in the proximal direction with respect to the main fixing portion 272 of the fixing portion 27, and the main fixing portion 272 and the second engagement portion 302 are in a non-engaged state.

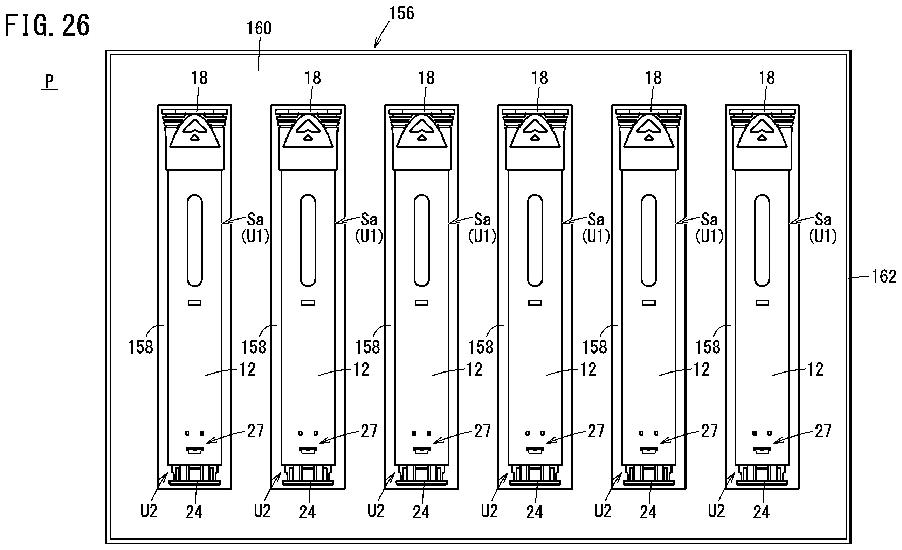

Before the liquid medicine administration device 10 containing the syringe 16 is assembled, the intermediate assembly Sa is housed in a packaging vessel 156 and is stored as a medical instrument package P as illustrated in Fig. 26. The medical instrument package P includes the intermediate assembly Sa of the liquid medicine administration device 10 and the packaging vessel 156 in which the intermediate assembly Sa is housed. The packaging vessel 156 includes a vessel main structure 160 having a housing portion 158. The vessel main structure 160 is formed in a substantially rectangular shape in plan view.

The vessel main structure 160 includes the housing portion 158 and an open end portion 162. The open end portion 162 is located at an upper end of the vessel main structure 160. An inside of the vessel main structure 160 is open through the open end portion 162. Note that the present invention is not limited to the case where the vessel main structure 160 is open. For example, the inside of the vessel main structure 160 may be sealed by providing a sealing film or the like at the open end portion 162 of the vessel main structure 160.

The housing portion 158 is recessed downward from the open end portion 162. A plurality of the housing portions 158 are provided in the vessel main structure 160 and are arranged in parallel in a longitudinal direction of the vessel main structure 160. Note that the present invention is not limited to the case where the plurality of the housing portions 158 are provided. The vessel main structure 160 may include one housing portion 158.

The intermediate assembly Sa is housed in each housing portion 158. A depth of the housing portion 158 with respect to the open end portion 162 is constant, and the intermediate assembly Sa is held substantially horizontally in the housing portion 158. The housing portion 158 is open on the open end portion 162 side. A longitudinal dimension of the housing portion 158 is equal to or larger than a longitudinal dimension of the intermediate assembly Sa.

Next, a case will be described, in which the liquid medicine administration device 10 is assembled using the intermediate assembly Sa (final assembly step).

First, as illustrated in Fig. 23, in a first step, the second unit U2 is pulled out from the first unit U1 of the intermediate assembly Sa in the proximal direction, whereby the first engagement protrusion 301A (the first engagement portion 301) is disengaged from the provisional fixing portion 271 of the fixing portion 27 in the proximal direction (the direction of the arrow A). Accordingly, the provisional fixing state between the first unit U1 and the second unit U2 is released. In this instance, the proximal inclined portion 3013 of the first engagement protrusion 301A comes into contact with an end portion of the provisional fixing portion 271 in the proximal direction, whereby the inner circumferential surface of the housing 12 is pushed and expanded radially outward, and the first engagement protrusion 301A is movable in the proximal direction while coming into contact with the inner circumferential surface of the housing 12 (see the two-dot chain line shape in Fig. 16).

After the distal end of the second unit U2 is released in the proximal direction (the direction of the arrow A) with respect to a proximal end of the first unit U1 in the first step, the syringe 16 is inserted into the housing 12 from a proximal portion of the first unit U1 in the distal direction in the second step from the state illustrated in Fig. 2. In this instance, the syringe 16 is inserted into the housing 12 from the protective cover 128 side. The syringe 16 is held by a syringe holder 19 housed in the housing 12 (see Fig. 5).

Next, in the third step, the second unit U2 is inserted into the housing 12 from the proximal portion of the first unit U1, and the second unit U2 is further pushed in the distal direction with respect to the provisional fixing position. Accordingly, as illustrated in Fig. 16, the first engagement portion 301 of the second unit U2 is moved in the distal direction beyond the fixing portion 27 (the provisional fixing portion 271) and is inserted into the reception portion 28 (the second hole 28A), and the second engagement portion 302 is inserted into the main fixing portion 272 of the fixing portion 27 while the inclined area 3022 is in sliding contact with the inner circumferential surface of the housing 12. In this instance, the inclined area 3022 facilitates insertion of the second engagement portion 302 into the housing 12. The latching portion 3021 of the second engagement portion 302 is latched by being engaged with a proximal portion of the main fixing portion 272. The first engagement portion 301 is inserted into the reception portion 28, whereby contact between the first engagement portion 301 and the housing 12 is avoided.

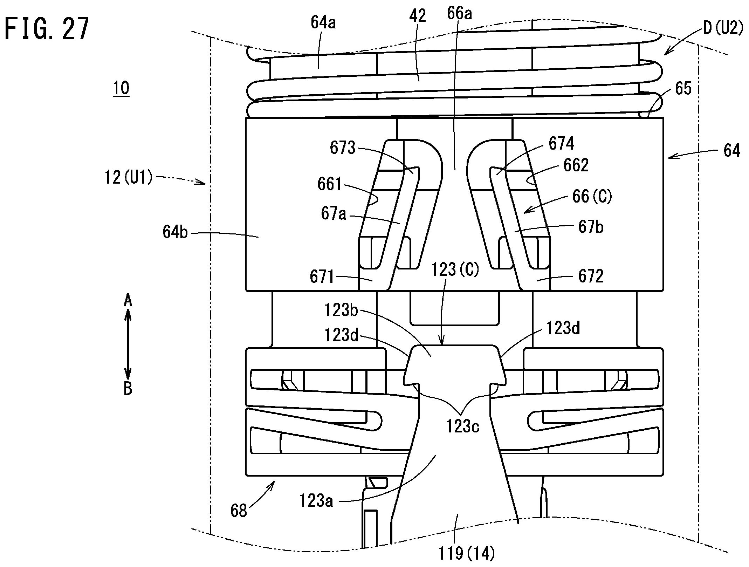

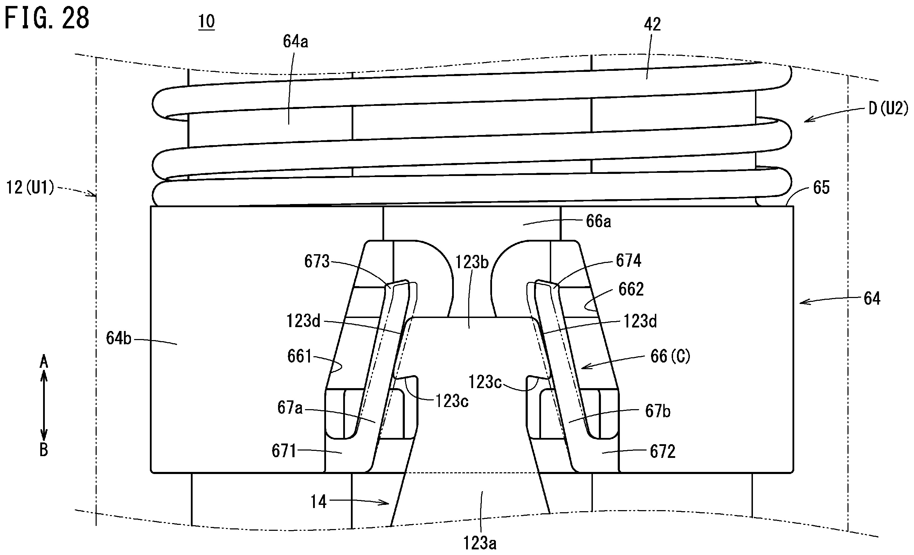

In the second unit U2, the proximal end of the drive unit D is moved toward the proximal end of the needle cover 14, and the first connection portions 66 and the second connection portions 123 face each other in the axial direction (the directions of the arrows A and B) as illustrated in Fig. 27. As illustrated in Fig. 28, when the drive unit D is further moved in the distal direction (the direction of the arrow B), the projection portion 123b of each second connection portion 123 is inserted into the housing recess 66a of each first connection portion 66 from the distal end of the cylindrical portion 64b. In each housing recess 66a, the projection portion 123b is inserted between the first flexible arm portion 67a and the second flexible arm portion 67b

The bevels 123d of the projection portion 123b come into contact with the first flexible arm portion 67a and the second flexible arm portion 67b, whereby the first flexible arm portion 67a and the second flexible arm portion 67b are pressed in directions away from each other along the circumferential direction of the cylindrical portion 64b. Accordingly, the first and second flexible arm portions 67a and 67b tilt to be separated from each other with the fixed ends 671 and 672 as fulcrums.

The projection portion 123b and the extension end 123a are inserted into each housing recess 66a while pushing and expanding the first and second flexible arm portions 67a and 67b in the circumferential direction of the cylindrical portion 64b. As illustrated in Fig. 11, when the projection portion 123b passes over the free ends 673 and 674 of the first and second flexible arm portions 67a and 67b, the first flexible arm portion 67a and the second flexible arm portion 67b tilt by a spring force in directions toward each other (see the two-dot chain line shape in Fig. 28). Accordingly, the projection portion 123b of each second connection portion 123 is engaged with proximal ends of the first and second flexible arm portions 67a and 67b, and the free ends 673 and 674 of the first and second flexible arm portions 67a and 67b are engaged with the eave portions 123c of the projection portion 123b. The first connection portions 66 of the sliding member 64 and the second connection portions 123 of the needle cover 14 are coupled in the axial direction (the directions of the arrows A and B) by each projection portion 123b, each first flexible arm portion 67a, and each second flexible arm portion 67b.

Accordingly, as illustrated in Fig. 1, main fixing of the second unit U2 to the fixing portion 27 of the first unit U1 is performed in a state where relative movement of the first unit U1 and the second unit U2 in the axial direction and relative movement thereof in the circumferential direction are restricted. In a state where the syringe 16 is housed in the first unit U1, an assembly completed state of the liquid medicine administration device 10 in which main fixing of the first unit U1 and the second unit U2 is completed is obtained. In the assembly completed state, in the liquid medicine administration device 10, the lid 76 of the end cap 24 abuts the proximal end of the housing 12 to close the proximal end.

In the assembly completed state of the liquid medicine administration device 10, the first engagement portion 301 is disengaged from the fixing portion 27 (the provisional fixing portion 271), and the second engagement portion 302 is engaged with the fixing portion 27 (the main fixing portion 272). In other words, the first engagement portion 301 is an engagement portion used to perform provisional fixing of the second unit U2 to the fixing portion 27 of the first unit U1 by being engaged with the fixing portion 27 before the syringe 16 is housed in the housing 12 of the first unit U1.

Next, a case will be described, in which the liquid medicine M is administered by the liquid medicine administration device 10.

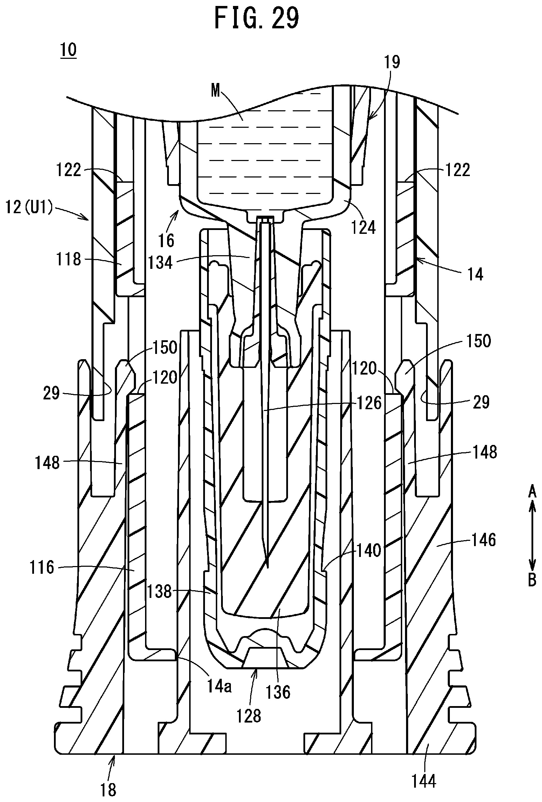

In the liquid medicine administration device 10 before use illustrated in Fig. 5, the cap 18 is detached from the distal ends of the housing 12 and the needle cover 14. In this instance, the patient grips the cap 18 of the housing 12 and pulls the cap 18 in a direction (the direction of the arrow B) away from the housing 12. Accordingly, as illustrated in Fig. 29, the cap 18 is moved in a direction away from the distal end of the needle cover 14, and the outer hooks 150 are moved in the distal direction (the direction of the arrow B) inside the engagement holes 120.

As the outer hooks 150 are moved in the distal direction, the outer hooks 150 are positioned to face the recesses 29. The cap 18 is further pulled in the distal direction (the direction of the arrow B). Accordingly, as illustrated in Fig. 12, the outer hooks 150 and the distal portions of the engagement holes 120 come into contact with each other, and the holding arms 148 tilt radially outward. The proximal ends of the holding arms 148 having the outer hooks 150 are moved into the recesses 29, whereby the holding arms 148 (the outer hooks 150) are disengaged from the engagement holes 120. Then, the holding arms 148 including the outer hooks 150 are moved in the distal direction via the recesses 29 and the gaps between the housing 12 and the needle cover 14.

As the cap 18 is moved toward the distal end, the protective cover 128 held by the inner hook 154 is moved together with the cap 18. Accordingly, the protective cover 128 is detached from the needle holding portion 134 of the barrel 124.

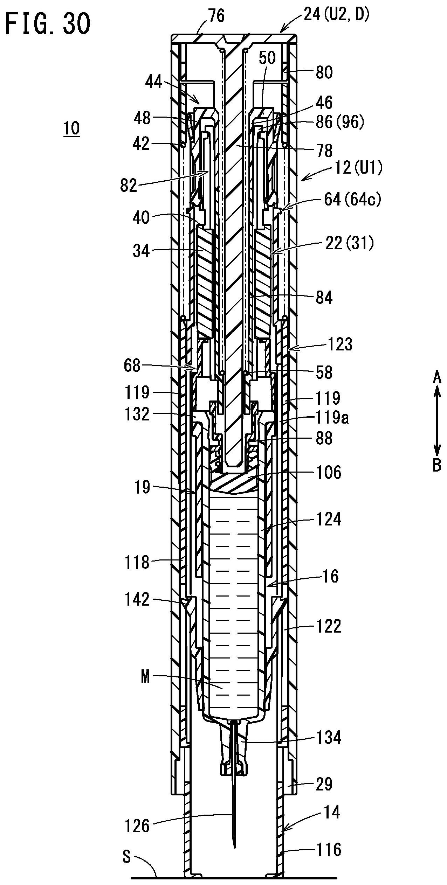

The engagement of the cap 18 with the needle cover 14 by the holding arms 148 is disengaged, and the cap 18 is fully detached from the distal end of the needle cover 14. Accordingly, as illustrated in Fig. 30, the distal ends of the housing 12 and the needle cover 14 are open, and simultaneously the protective cover 128 that has been covering the puncture needle 126 is detached.

Next, the liquid medicine M is administered using the liquid medicine administration device 10 from which the cap 18 has been detached.

In a state before puncturing is performed by the liquid medicine administration device 10, a relationship between the plunger 82 and the locking pin 44 is as illustrated in Fig. 18. The locking pin 44 is inserted into the flexible portions 86 of the plunger 82, and the flexible portions 86 are restricted from tilting inward by the locking pin 44. In other words, the flexible portions 86 cannot tilt inward. Since the flanges 96 of the flexible portions 86 are engaged with the protrusion portions 38 of the sleeve body 22, the plunger 82 cannot be moved in the distal direction.

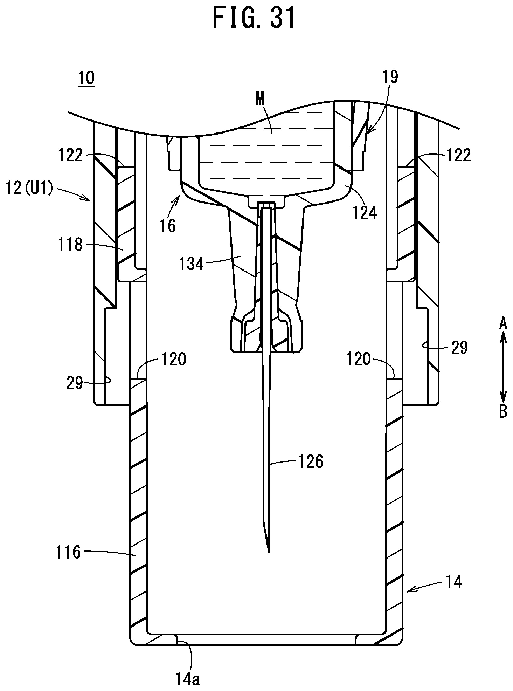

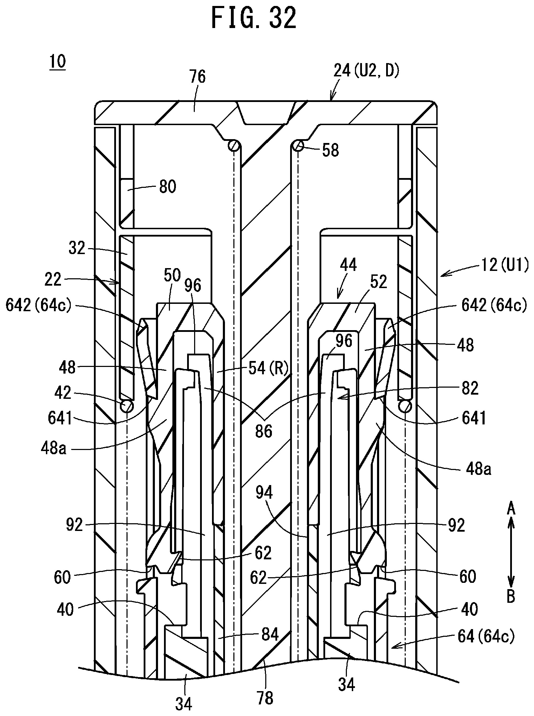

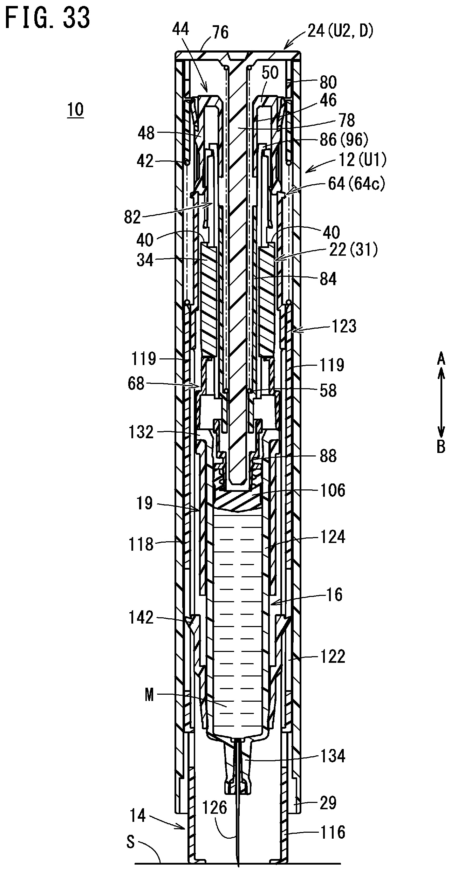

As illustrated in Fig. 30, the patient grips the housing 12 of the liquid medicine administration device 10 and presses, at a substantially right angle, the distal end of the needle cover 14 projecting from the distal end of the housing 12 against skin S that becomes a desired puncture site. In this instance, the distal side and the proximal side of the liquid medicine administration device 10 are in states illustrated in Figs. 31 and 32, respectively. Next, as illustrated in Fig. 33, the housing 12 is continuously pushed against the skin S (the distal side, the direction of the arrow B). As the housing 12 is pushed against the skin S, the skin S pushes the needle cover 14, and the needle cover 14 is moved relative to the housing 12 in the proximal direction against an elastic force of the sleeve spring 42. Fig. 33 illustrates a state where a needle tip of the puncture needle 126 has just started to touch the skin S.

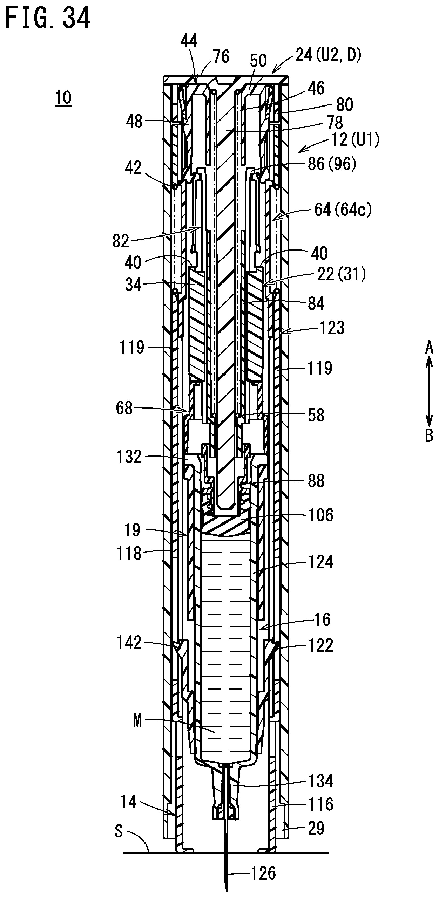

As the housing 12 is further pushed against the skin S, the puncture needle 126 of the syringe 16 projects from the hole portion 14a of the needle cover 14 toward the distal side (the direction of the arrow B) as illustrated in Fig. 34. Accordingly, the state is changed to a puncture completed state where the puncture needle 126 pierces the skin S and is inserted to a predetermined depth. In the puncture completed state, the distal end of the housing 12 is substantially at the same position as the distal end of the needle cover 14.

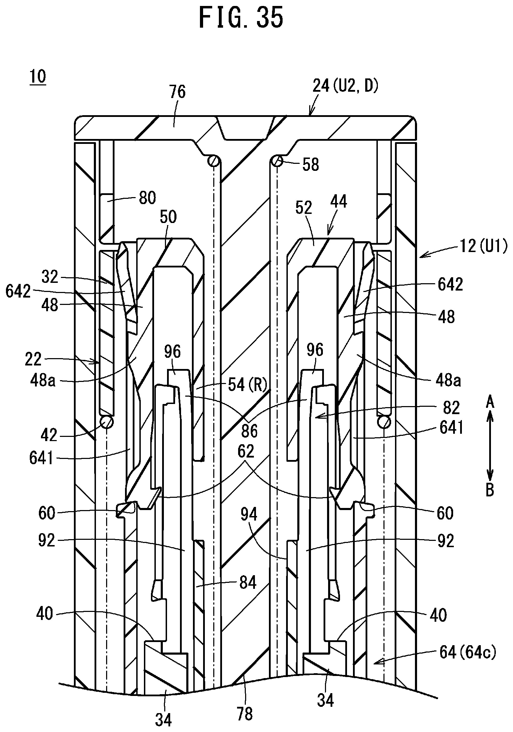

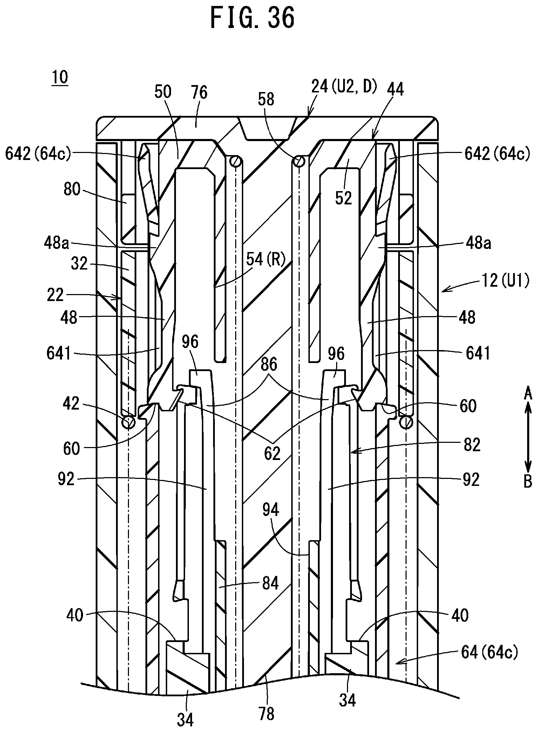

The ribs 119a of the needle cover 14 abut the distal end of the sliding member 64. Therefore, in the process of state change from Fig. 30 to Fig. 34, as the needle cover 14 is moved in the proximal direction relatively to the housing 12 as described above, the sliding member 64 is also moved in the proximal direction relative to the housing 12. As illustrated in Figs. 35 and 36, as the sliding member 64 is moved in the proximal direction, the distal ends of the slit holes 641 of the sliding member 64 push, in the proximal direction, the engagement end 60 provided at the distal end of the arm portion 48 of the locking pin 44, so that the locking pin 44 is also moved in the proximal direction relatively to the housing 12.

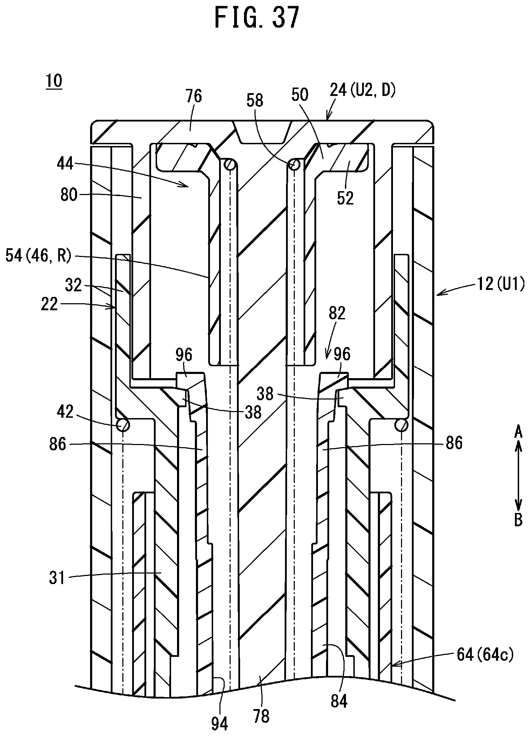

As the locking pin 44 is moved in the proximal direction (the direction of the arrow A) relatively to the housing 12, the small diameter portion 54 of the locking pin 44 is separated from an inner side of the flexible portions 86 of the plunger 82 in the proximal direction (the direction of the arrow A) as illustrated in Fig. 37. Accordingly, the locked state of the plunger 82 by the locking pin 44 is cancelled, and the plunger 82 starts moving in the distal direction (the direction of the arrow B) by the spring force of the injection spring 58.

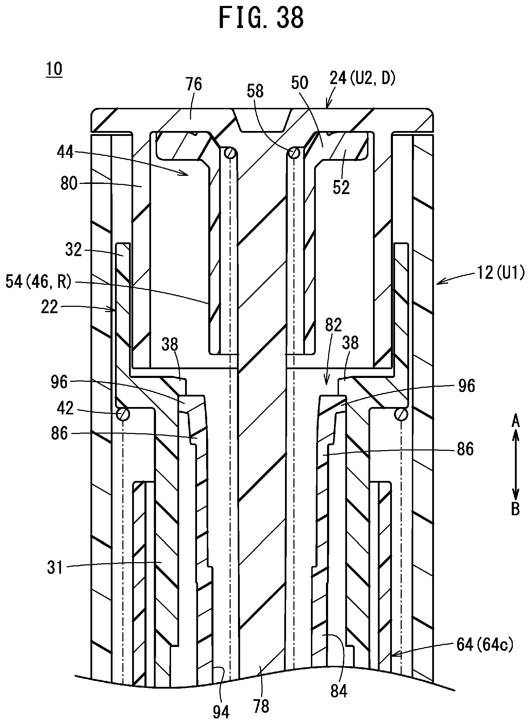

When the plunger 82 starts moving in the distal direction from the state illustrated in Fig. 37, the four flanges 96 are pushed radially inward by the contact between tapered distal surfaces thereof and the protrusion portions 38. Accordingly, each flexible portion 86 tilts radially inward with respect to the pin main structure 46. As illustrated in Fig. 38, when the plunger 82 is further moved in the distal direction, the flanges 96 pass over the protrusion portions 38 and is moved in the distal direction.

When the flanges 96 of the plunger 82 pass over the protrusion portions 38, the flanges 96 are displaced radially inward, and then, expand radially outward again by an elastic restoring force of the flexible portions 86, and outer edge portions come into contact with the inner circumferential surface of the body main structure 31. In this instance, since the outer diameter D1 of the flanges 96 is slightly larger than the inner diameter D2 of the body main structure 31 as illustrated in Fig. 19, a first notification sound that is a contact sound (a click sound) is generated when the flanges 96 are brought into contact with the body main structure 31.

After the plunger 82 starts moving in the distal direction, and the flanges 96 pass over the protrusion portions 38, the plunger 82 is further moved in the distal direction, thereby the plunger 82 starting to press the gasket 106. The liquid medicine M in the barrel 124 is pressed toward the distal end by the gasket 106. Accordingly, the liquid medicine M is ejected from the puncture needle 126, and the liquid medicine M starts to be subcutaneously administered to the patient. As described above, when the plunger 82 starts moving in the distal direction, and the flanges 96 pass over the protrusion portions 38, the first notification sound is generated as the flanges 96 collide with the body main structure 31. When the patient recognizes the first notification sound, the patient can confirm that the liquid medicine M starts to be administered.

When the plunger 82 is moved in the distal direction, the plunger 82 is guided by each guide rib 34 of the sleeve body 22 inserted into the four sliding grooves 92. Accordingly, when the plunger 82 is moved toward the distal end, the plunger 82 is moved only along the axial direction without rotating. The gasket 106 mounted on the top member 88 is inserted into the barrel 124 of the syringe 16.

After the administration of the liquid medicine M is started, the plunger 82 continues moving in the distal direction (the direction of the arrow B) at a constant speed continuously by the spring force of the injection spring 58. The liquid medicine M is pushed out by the gasket 106 moving in the distal direction inside the barrel 124, and the liquid medicine M is ejected from the puncture needle 126. The plunger 82 is further moved in the distal direction, thereby causing the flexible portions 86 to reach a proximal end of the end guide 68.

As illustrated in Fig. 21A, when the flanges 96 are brought into contact with the inclined guide portions 72, the flanges 96 are moved in the distal direction (the direction of the arrow B) while rotating clockwise along the inclined guide portions 72. In other words, when the plunger 82 is moved along the inclined guide portions 72 of the end guide 68, a rotational force is applied to the plunger 82.

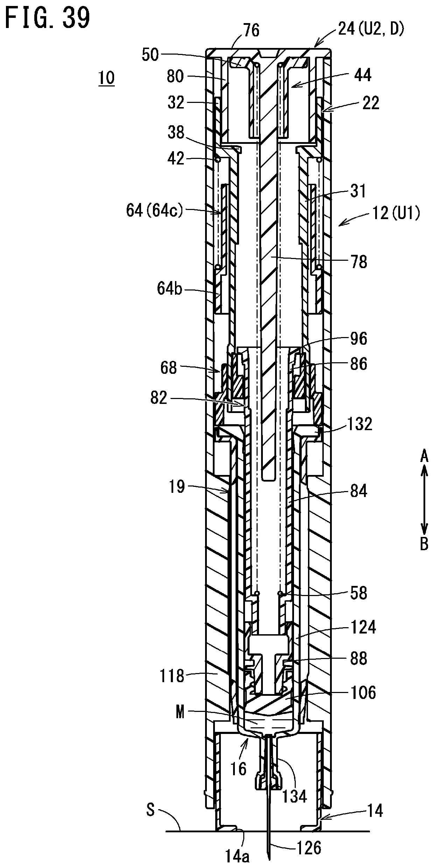

Simultaneously, as illustrated in Fig. 21B, the rotation of the plunger 82 causes the engagement pieces 98 provided at the distal end to be moved along the horizontal portion 110 in the guide grooves 108 of the top member 88. Note that, as illustrated in Fig. 39, the plunger 82 reaches the distal ends of the guide ribs 34 of the sleeve body 22, a guided state in the axial direction by the sliding grooves 92 and the guide ribs 34 is released, and the plunger 82 becomes rotatable.

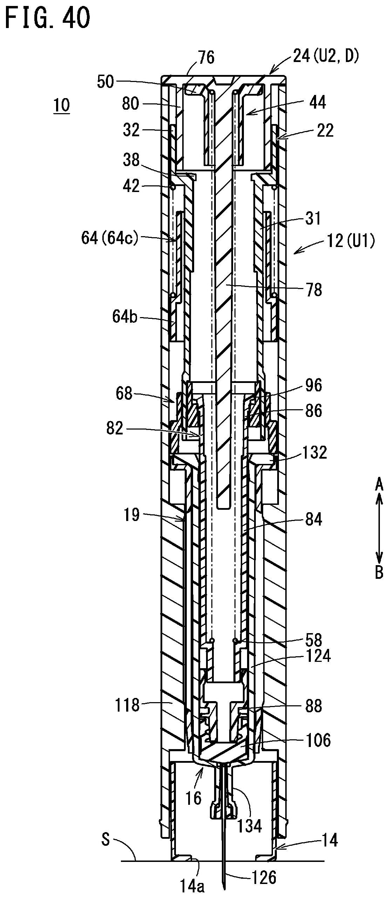

As illustrated in Fig. 22A, the flexible portions 86 are moved in the distal direction while rotating along the inclined guide portion 72 and reach the straight guide portions 74. In this instance, as illustrated in Fig. 40, the plunger 82 administers a predetermined amount of the liquid medicine M in the barrel 124 to the skin S through the puncture needle 126 and ends the administration of the liquid medicine M.

Before the administration of the liquid medicine M is ended, as illustrated in Fig. 22B, the engagement pieces 98 of the rotating plunger 82 reach the inclined portions 114 of the guide grooves 108. The guide grooves 70 function as a guide means for moving the plunger 82 straightly in the distal direction (the direction of the arrow B) after rotating the plunger 82 at a predetermined degree.

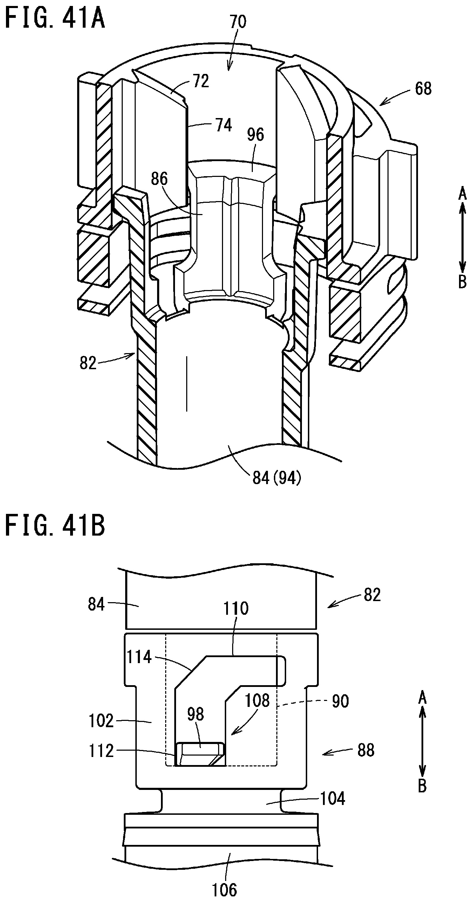

After the administration of the liquid medicine M is ended, the engagement pieces 98 of the plunger 82 are moved toward the perpendicular portions 112 while rotating along the inclined portions 114. The engagement pieces 98 of the plunger 82 are moved along the perpendicular portions 112, thereby moving again in the axial direction toward the distal direction (the direction of the arrow B). As illustrated in Figs. 41A and 41B, distal surfaces of the engagement pieces 98 moved along the perpendicular portions 112 of the guide grooves 108 are brought into contact with distal edges of the perpendicular portions 112, whereby a second notification sound that is a contact sound (a click sound) is generated.

The second notification sound is generated in a predetermined time after the administration of the liquid medicine M is ended. The second notification sound enables the patient to recognize the end of liquid medicine administration to an affected portion (skin S). After the patient recognizes the second notification sound, the patient separates the liquid medicine administration device 10 from the affected portion.

In other words, after the liquid medicine administration by the liquid medicine administration device 10 is ended, the plunger 82 is rotated by the guide grooves 70 of the end guide 68, and a moving speed in the axial direction is slowed. Accordingly, a time taken for the gasket 106 of the top member 88 connected to the plunger 82 to reach the distal end of the barrel 124 is delayed. After a predetermined time from the end of the liquid medicine administration, the second notification sound for notifying the patient that the liquid medicine administration is ended is generated.

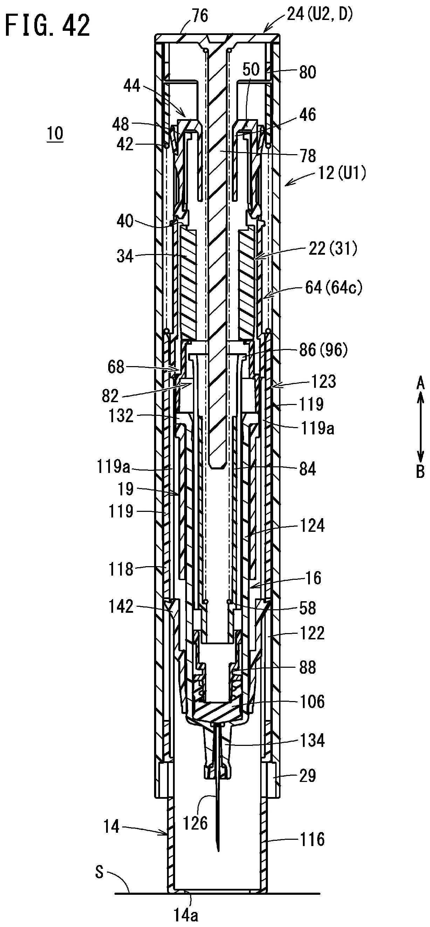

When the patient cancels a pressing force of the liquid medicine administration device 10 against the skin S, the sliding member 64 is biased toward the distal end by a spring force of the sleeve spring 42 as illustrated in Fig. 42. Since the distal end of the sliding member 64 is brought into contact with the ribs 119a of the needle cover 14, the sliding member 64 pushes the needle cover 14 in the distal direction and is moved together with the needle cover 14 in the distal direction relatively to the housing 12. The needle cover 14 is moved to a position where the distal end of the needle cover 14 is positioned in the distal direction with respect to the puncture needle 126. Accordingly, the puncture needle 126 is fully covered by the needle cover 14.

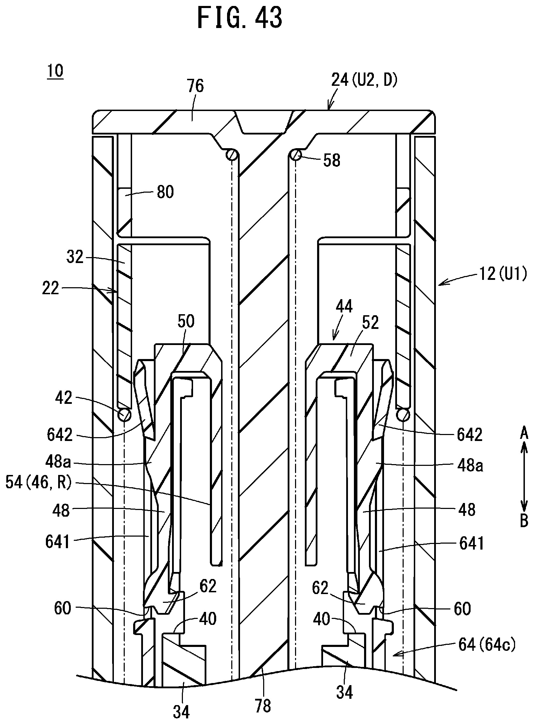

As illustrated in Fig. 43, since the projecting pieces 642 of the sliding member 64 are engaged with the protruding portions 48a of the locking pin 44, the locking pin 44 is moved in the distal direction (the direction of the arrow B) together with the sliding member 64. As the locking pin 44 is moved in the distal direction, the locking claws 62 are engaged with the locking grooves 40 of the sleeve body 22. Accordingly, the movement of the locking pin 44 in the proximal direction with respect to the sleeve body 22 is restricted. The sliding member 64 abuts the arm portions 48 of the locking pin 44, and the movement of the sliding member 64 in the proximal direction is also restricted. The extension portions 119 (the ribs 119a) of the needle cover 14 abut the distal end of the sliding member 64, and the movement of the needle cover 14 in the proximal direction is also restricted.

Since the movement of the needle cover 14 in the proximal direction is restricted, the puncture needle 126 is secured and is not exposed to the outside by the needle cover 14 even in a case where a force in the proximal direction acts on the needle cover 14.

The embodiments have the following effects.

As illustrated in Fig. 3, the intermediate assembly Sa of the liquid medicine administration device 10 includes the first unit U1 and the second unit U2 attached to the first unit U1. In the state where the syringe 16 is not housed in the first unit U1, provisional fixing of the second unit U2 to the fixing portion 27 of the first unit U1 is performed. In the state where the syringe 16 is housed in the housing 12 of the first unit U1, main fixing of the second unit U2 and the first unit U1 is performed. Accordingly, as compared with a case where a first member, a second member, and a syringe filled with a liquid medicine form respective units (assemblies), and the three assemblies are provided in a final assembly step, the number of assemblies to be supplied in the final assembly step can be reduced by using the intermediate assembly Sa in which the first and second units U1 and U2 are provisionally fixed, and an assembly step of the liquid medicine administration device 10 can be simplified.

As illustrated in Fig. 6, the second unit U2 has the first engagement portion 301 for provisional fixing and the second engagement portion 302 for main fixing. As illustrated in Fig. 17, in the state where the first unit U1 and the second unit U2 are provisionally fixed, the first engagement portion 301 is engaged with the fixing portion 27. As illustrated in Fig. 6, in the state where main fixing of the first unit U1 and the second unit U2 is performed, the second engagement portion 302 is engaged with the fixing portion 27. Accordingly, provisional fixing of the first unit U1 and the second unit U2 can be performed by engaging the first engagement portion 301 with the fixing portion 27, and main fixing of the first unit U1 and the second unit U1 can be effectively performed by engaging the second engagement portion 302 with the fixing portion 27.

The fixing portion 27 includes the provisional fixing portion 271 and the main fixing portion 272 provided at a position different from a position of the provisional fixing portion 271. In the state where the first unit U1 and the second unit U2 are provisionally fixed, the first engagement portion 301 is engaged with the provisional fixing portion 271. In the state where main fixing of the first unit U1 and the second unit U2 is completed, the second engagement portion 302 is engaged with the main fixing portion 272. Accordingly, provisional fixing of the first unit U1 and the second unit U2 can be performed by engaging the first engagement portion 301 with the provisional fixing portion 271, and main fixing of the first unit U1 and the second unit U2 can be effectively performed by engaging the second engagement portion 302 with the main fixing portion 272.

The fixing portion 27 may be a first hole 27A (a recess) formed in the housing 12, and the provisional fixing portion 271 and the main fixing portion 272 may be formed in different areas of the first hole 27A. Accordingly, the provisional fixing portion 271 and the main fixing portion 272 can be provided in the same first hole 27A, so that the provisional fixing portion 271 and the main fixing portion 272 can be easily formed.

The main fixing portion 272 is provided in the proximal direction with respect to the provisional fixing portion 271 in the axial direction of the housing 12. Accordingly, by moving the second unit U2 in the distal direction, the first engagement portion 301 can be disengaged from the provisional fixing portion 271 in the distal direction, and the second engagement portion 302 can be engaged with the main fixing portion 272.

The fixing portion 27 has the first region F1 in which the provisional fixing portion 271 is provided and the second region F2 which is adjacent to the proximal side of the first region F1 in the axial direction of the housing 12 and in which the main fixing portion 272 is provided. The first region F1 is formed to be wider than the second region F2 in the circumferential direction of the housing 12. Accordingly, the provisional fixing portion 271 and the main fixing portion 272 can be easily provided in one first hole 27A.

As illustrated in Fig. 2, the first engagement portion 301 has the first engagement protrusion 301A that is provided on the outer circumferential surface of the sleeve body 22 and is engageable with the fixing portion 27, and the second engagement portion 302 has the second engagement protrusion 302A that is provided on the end cap 24 attached to the proximal end of the sleeve body 20 and is engageable with the fixing portion 27. Accordingly, provisional fixing of the first unit U1 and the second unit U2 can be performed by engaging the first engagement protrusion 301A provided on the sleeve body 22 with the fixing portion 27, and main fixing of the first unit U1 and the second unit U2 can be effectively performed by engaging the second engagement protrusion 302A provided on the end cap 24 with the fixing portion 27.

As illustrated in Figs. 7A and 7B, the plurality of the fixing portions 27 are provided in the circumferential direction of the housing 12, and the plurality of the first engagement portions 301 and the plurality of the second engagement portions 302 are individually provided in the circumferential direction of the second unit U2. Accordingly, when the second unit U2 is inserted into and provisionally fixed to the first unit U1, the first engagement portion 301 and the second engagement portion 302 are easily aligned with the fixing portion 27 in the circumferential direction of the housing 12 and the second unit U2.

As illustrated in Fig. 17, the housing 12 has the reception portion 28 formed in the second hole 28A located in the distal end direction with respect to the fixing portion 27. The first engagement portion 301 is inserted into the reception portion 28 when main fixing of the first unit U1 and the second unit U2 is completed by the main fixing portion 272. Accordingly, in the state where the first unit U1 and the second unit U2 are fixed, the first engagement portion 301 moved in the distal direction from the fixing portion 27 is inserted into the reception portion 28, so that interference between the first engagement portion 301 and the housing 12 is effectively prevented.

As illustrated in Fig. 6, the first engagement portion 301 for provisional fixing is the engagement portion used to perform provisional fixing of the second unit U2 to the fixing portion 27 of the first unit U1 by being engaged with the fixing portion 27 before the syringe 16 is housed in the housing 12 of the first unit U1. In the assembly completed state where the syringe 16 is housed in the first unit U1, and main fixing of the second unit U2 to the fixing portion 27 is completed, the first engagement portion 301 is disengaged from the fixing portion 27, and the second engagement portion 302 is engaged with the fixing portion 27. Accordingly, the number of units supplied in the assembly step can be reduced by using the intermediate assembly Sa in which the first and second units U1 and U2 are provisionally fixed, and an assembly step of the liquid medicine administration device 10 can be simplified, as compared with a configuration in which a first member, a second member, and a syringe filled with a liquid medicine form respective units, and the three units are finally assembled.

The method includes: the first step of detaching the second unit U2 from the fixing portion 27 of the first unit U1 of the intermediate assembly Sa and disengaging the second unit U2 from the first unit U1 in the proximal direction; the second step of inserting the syringe 16 into the housing 12; and the third step of inserting the second unit U2 into the housing 12 and performing main fixing of the second unit U2 to the fixing portion 27 of the first unit U1. Accordingly, the number of components supplied in the assembly step can be reduced by using the intermediate assembly Sa in which the first and second units U1 and U2 are provisionally fixed, and an assembly step of the liquid medicine administration device 10 can be simplified, as compared with the configuration in which the first member, the second member, and the syringe filled with the liquid medicine form respective units, and the three units are finally assembled.

The second unit U2 has the first engagement portion 301 for provisional fixing and the second engagement portion 302 for main fixing. In the first step, the second unit U2 is detached, from the provisional fixing state where the first engagement portion 301 is engaged with the fixing portion 27, by disengaging the first engagement portion 301 from the fixing portion 27. In the third step, the second unit U2 is brought into the main fixing state with respect to the first unit U1 by engaging the second engagement portion 302 with the fixing portion 27.

Accordingly, provisional fixing of the first unit U1 and the second unit U2 can be performed by engaging the first engagement portion 301 with the fixing portion 27, and main fixing of the first unit U1 and the second unit U2 can be effectively performed in the state where the syringe 16 is housed by engaging the second engagement portion 302 with the fixing portion 27.

As illustrated in Fig. 26, since the medical instrument package P includes the intermediate assembly Sa of the liquid medicine administration device 10 and the packaging vessel 156 housing the intermediate assemblies Sa, the intermediate assembly Sa in which the first and second units U1 and U2 are provisionally fixed can be integrally housed in the packaging vessel 156, as compared with the case where the first unit U1 and the second unit U2 are individually housed in the packaging vessels 156, respectively. Therefore, space saving of a storage place of the intermediate assembly Sa can be achieved.

Note that the present invention is not limited to the above-described disclosure and can employ various configurations without departing from the gist of the present invention.

## Claims

1. An intermediate assembly of a liquid medicine administration device, comprising: a first unit; and a second unit attached to the first unit, wherein
the first unit has a hollow cylindrical housing and enables a syringe filled with a liquid medicine to be housed in the housing,
the second unit has a drive unit biasing a gasket of the syringe toward a distal end of the housing and is attached to a fixing portion provided at a proximal portion of the housing of the first unit, and at least a part of the second unit is housed in the housing,
in a state where the syringe is not housed in the first unit, provisional fixing of the second unit to the fixing portion of the first unit is performed, and
in a state where the second unit is detached from the fixing portion of the first unit so that a provisional fixing state is released, and the syringe is housed in the housing of the first unit, main fixing of the second unit and the first unit is able to be performed.

2. The intermediate assembly of a liquid medicine administration device according to claim 1, wherein
the second unit has a first engagement portion for provisional fixing and a second engagement portion for main fixing,
in a state where the first unit and the second unit are provisionally fixed, the first engagement portion is engaged with the fixing portion, and
in a state where the provisional fixing state between the first unit and the second unit is released, and main fixing of the first unit and the second unit is completed, the first engagement portion is disengaged from the fixing portion, and the second engagement portion is engaged with the fixing portion.

3. The intermediate assembly of a liquid medicine administration device according to claim 2, wherein
the fixing portion includes a provisional fixing portion and a main fixing portion provided at a position different from a position of the provisional fixing portion,
in a state where the first unit and the second unit are provisionally fixed, the first engagement portion is engaged with the provisional fixing portion, and
in a state where main fixing of the first unit and the second unit is completed, the first engagement portion is disengaged from the provisional fixing portion, and the second engagement portion is engaged with the main fixing portion.

4. The intermediate assembly of a liquid medicine administration device according to claim 3, wherein
the fixing portion is a hole or a recess formed in the housing, and
the provisional fixing portion and the main fixing portion are formed in different areas of the hole or the recess.

5. The intermediate assembly of a liquid medicine administration device according to claim 4, wherein
the main fixing portion is provided on a proximal side of the housing in an axial direction with respect to the provisional fixing portion.

6. The intermediate assembly of a liquid medicine administration device according to claim 5, wherein
the fixing portion has a first region in which the provisional fixing portion is provided and a second region which is adjacent to a proximal side of the first region in the axial direction of the housing and in which the main fixing portion is provided, and
the first region is formed to be wider than the second region in a circumferential direction of the housing.

7. The intermediate assembly of a liquid medicine administration device according to claim 2, wherein
the fixing portion is a hole or a recess formed in the housing,
the first engagement portion has a first engagement protrusion that is provided on an outer circumferential surface of a sleeve body and is engageable with the fixing portion, the sleeve body being attached to the proximal portion of the housing and being housed in the housing, and
the second engagement portion has a second engagement protrusion that is provided on an end cap attached to a proximal end of the sleeve body and is engageable with the fixing portion.

8. The intermediate assembly of a liquid medicine administration device according to any one of claims 2 to 7, wherein
a plurality of the fixing portions are provided in the circumferential direction of the housing, and
a plurality of the first engagement portions and a plurality of the second engagement portions are individually provided in a circumferential direction of the second unit.

9. The intermediate assembly of a liquid medicine administration device according to any one of claims 3 to 6, wherein
the housing has a reception portion formed in a hole or a recess located in a distal direction with respect to the fixing portion, and
the first engagement portion is inserted into the reception portion when the first unit and the second unit are fixed by the main fixing portion.

10. A liquid medicine administration device comprising:
a first unit having a hollow cylindrical housing;
a syringe housed in the housing and filled with a liquid medicine; and
a second unit which has a drive unit biasing a gasket of the syringe toward a distal end of the housing and is attached to the first unit, and at least a part of which is housed in the housing, wherein
a proximal portion of the housing of the first unit has a fixing portion,
the second unit has a first engagement portion for provisional fixing and a second engagement portion for main fixing,
the first engagement portion is an engagement portion used to perform provisional fixing of the second unit to the fixing portion of the first unit by being engaged with the fixing portion before the syringe is housed in the housing of the first unit, and
in an assembly completed state where the syringe is housed in the housing, and main fixing of the second unit to the fixing portion of the first unit is completed, the first engagement portion is disengaged from the fixing portion, and the second engagement portion is engaged with the fixing portion.

11. A method for assembling a liquid medicine administration device using the intermediate assembly according to claim 1, the method comprising:
a first step of detaching the second unit from the fixing portion of the first unit of the intermediate assembly and disengaging the second unit from the first unit in a proximal direction;
a second step of inserting the syringe into the housing in a distal direction from the proximal portion of the first unit and housing the syringe in the housing; and
a third step of inserting the second unit into the housing from the proximal portion of the first unit and performing main fixing of the second unit to the fixing portion of the first unit.

12. The method for assembling a liquid medicine administration device according to claim 11, wherein
the second unit has a first engagement portion for provisional fixing and a second engagement portion for main fixing,
in the first step, the second unit is detached, from a provisional fixing state where the first engagement portion is engaged with the fixing portion, by disengaging the first engagement portion from the fixing portion, and
in the third step, the second unit is brought into a main fixing state with respect to the first unit by engaging the second engagement portion with the fixing portion.

13. A medical instrument package comprising:
the intermediate assembly of a liquid medicine administration device according to claim 1; and
a packaging vessel in which the intermediate assembly is housed.
